# EUROPÄISCHE PATENTSCHRIFT

(11) **EP 3 297 581 B1**
(45) Veröffentlichungstag und Bekanntmachung des Hinweises auf die Patenterteilung: **03.07.2019**
(21) Anmeldenummer: 16728227.6
(22) Anmeldetag: 18.05.2016
(51) Int. Cl.: A61F 2/68, A61F 2/64, A61F 2/70, F16F 9/53, A61F 2/50, A61F 2/76

(54) **PROTHESEN- ODER EXOSKELETTKOMPONENTE UND VERFAHREN**
PROSTHETIC OR EXOSKELETON COMPONENT AND METHOD
ÉLÉMENTS DE PROTHÈSE OU D'EXOSQUELETTE ET PROCÉDÉ

(30) Priorität: 18.05.2015 DE 102015107783
(43) Veröffentlichungstag der Anmeldung: 28.03.2018
(73) Patentinhaber: Inventus Engineering GmbH, 6771 St. Anton i. M. (AT)
(72) Erfinder: BATTLOGG, Stefan, 6771 St. Anton i.M. (AT)
(74) Vertreter: BSB Patentanwälte Schütte & Engelen Partnerschaft mbB
(86) Internationale Anmeldenummer: PCT/EP2016/061148
(87) Internationale Veröffentlichungsnummer: WO 2016/184919

(56) Entgegenhaltungen:
- WO-A1-2006/069264
- US-A1- 2010 305 716
- US-B1- 6 423 098
- US-B2- 7 985 265
- Bernhard Kleiner ET AL: "Foresighted control of active foot prostheses", Proceedings SENSOR 2011, 31 December 2011 (2011-12-31), pages 669-672, XP055534016, DOI: 10.5162/sensor11/d8.4 ISBN: 978-3-9810993-9-3

## Beschreibung

Die vorliegende Erfindung betrifft eine Prothesenkomponente oder Exoskelettkomponente für eine Prothese oder Exoskelett mit wenigstens einer Stoßdämpfereinrichtung und ein Verfahren zum Betreiben einer Prothese oder einem Exoskelett. Die Stoßdämpfereinrichtung umfasst wenigstens eine über wenigstens eine Steuereinrichtung steuerbare Dämpfereinrichtung.

Die Dämpfung hat einen großen Einfluss auf die Bewegungseigenschaften und stellt daher ein wichtiges Merkmal einer Prothese oder eines Exoskeletts und insbesondere von Arm, Bein-, Hüft- oder Fußprothesen dar. So ermöglichen Stoßdämpfer ein verbessertes Gehbild und erlauben ein zügiges Gehen selbst im Gelände. Üblicherweise umfasst ein Stoßdämpfer auch eine Federeinheit, um auftretende Stöße abzufedern und eine Dämpfungseinheit, um die Schwingung zu dämpfen.

Um die Vorteile einer Stoßdämpfung optimal ausnutzen zu können, ist in der Regel eine Einstellung der Dämpfungs- und Federeigenschaften unerlässlich. Kriterien für die Einstellung sind dabei beispielsweise das Gewicht des Trägers und dessen Bewegungsgewohnheiten sowie die Eigenschaften des Geländes, in dem gegangen werden soll.

Dazu ist in der Regel eine Justierung des Stoßdämpfers nötig, bei der eine Reihe von Parametern für Dämpfung und Federung eingestellt und aufeinander abgestimmt werden müssen. Besonders für Anfänger oder ältere Personen ist eine solche Einstellung jedoch nicht immer unproblematisch bzw. diese vergessen diese. In Extremfällen ist es sogar möglich, dass durch eine Kombination ungünstiger Einstellungen die Trageigenschaften verschlechtert werden.

Im Stand der Technik sind daher Stoßdämpfer bekannt geworden, welche speziell für Anfänger nur wenige oder nur die wichtigsten Einstellmöglichkeiten zur Verfügung stellen. Im Gegensatz dazu können Stoßdämpfer für fortgeschrittene Benutzer oder Experten eine größere Zahl von Einstellmöglichkeiten aufweisen. Ein typischer Träger von Prothesen oder Exoskeletten ist dadurch aber in der Regel überfordert.

Mit der US 7,985,265 B2 ist eine Prothese mit den Merkmalen des Oberbegriffs von Anspruch 1 bekannt geworden.

Es ist die Aufgabe der vorliegenden Erfindung, eine Prothesen- oder Exoskelettkomponente zur Verfügung zu stellen, welche die Anpassung von Dämpfungseigenschaften insbesondere während der Bewegung vereinfacht.

Diese Aufgabe wird durch eine Prothesen- oder Exoskelettkomponente mit den Merkmalen des Anspruchs 1, sowie durch ein Verfahren nach Anspruch 14 gelöst. Bevorzugte Weiterbildungen der Erfindung sind Gegenstand der Unteransprüche. Weitere Vorteile und Merkmale ergeben sich durch die allgemeine Beschreibung und die Beschreibung der Ausführungsbeispiele.

Eine erfindungsgemäße Prothesen- oder Exoskelettkomponente für eine Prothese oder ein Exoskelett umfasst wenigstens eine Stoßdämpfereinrichtung mit wenigstens einer über wenigstens eine Steuereinrichtung steuerbaren Dämpfereinrichtung. Es ist wenigstens eine Erkennungseinrichtung mit wenigstens einer Sensoreinheit vorgesehen, welche wenigstens eine Empfangseinheit zur berührungslosen Erfassung wenigstens eines Signals und insbesondere eines von wenigstens einer Unwegsamkeit beeinflussten Signals umfasst. Die Erkennungseinrichtung ist dazu geeignet und ausgebildet, in Abhängigkeit des erfassten Signals die Unwegsamkeit zu erkennen und die oder wenigstens eine Dämpfereinrichtung oder die Stoßdämpfereinrichtung in Abhängigkeit der erkannten Unwegsamkeit zu steuern, sodass wenigstens eine Dämpfungseigenschaft der Dämpfereinrichtung durch ein Signal der Erkennungseinrichtung einstellbar ist. Die Sensoreinheit umfasst wenigstens eine Sendeeinheit zur Aussendung wenigstens eines Signals und die Empfangseinheit ist dazu geeignet und ausgebildet, wenigstens eine von der Unwegsamkeit ausgehende Reflexion des ausgesendeten Signals als Signal zu erfassen

Die erfindungsgemäße Prothesen- oder Exoskelettkomponente hat viele Vorteile. Ein erheblicher Vorteil ist, dass Unwegsamkeiten in Form von Hindernissen, Störstellen, Treppen etc. erkannt werden können und dass die Dämpfereinrichtung in Folge der erkannten Unwegsamkeit eingestellt wird. Dadurch ist eine sehr einfache und komfortable Anpassung der Dämpfungseigenschaften an die jeweils vorherrschenden Geh-, Straßen- oder Geländebedingungen möglich. Beispielsweise kann der Träger direkt von einem ebenen Gehweg auf einen anspruchsvollen Pfad mit zahlreichen Unebenheiten und Stufen wechseln, ohne, dass er sich Gedanken über eine Umstellung der Dämpfungseigenschaften machen muss oder eine (starke) Verschlechterung der Gehbewegung eintritt.

Ein weiterer Vorteil ist, dass die Stärke der Dämpfung ganz gezielt auf eine bestimmte, demnächst zu erreichende Unwegsamkeit eingestellt werden kann. So können beispielsweise kleinere Unebenheiten weniger stark gedämpft werden als größere Stufen im Gelände. Eine solche Anpassung der Dämpfungseigenschaften an das aktuell vorherrschende Gelände ermöglicht auch eine optimale Ausnutzung der maximal erzielbaren Dämpfung eines Stoßdämpfers, sodass die Prothese selbst bei einer extremen Bewegung stets angepasst ist. Durch die bessere Ausnutzung können die Stoßdämpfer zudem kleiner dimensioniert werden, sodass auch Gewicht eingespart wird.

Die Erkennungseinrichtung ist insbesondere dazu geeignet und ausgebildet, die Unwegsamkeit anhand des erfassten Signals wenigstens teilweise zu charakterisieren. Eine solche Charakterisierung ist beispielsweise die Bestimmung einer Form bzw. Geometrie der Unwegsamkeit und/oder eine Einordnung der Unwegsamkeit in eine hinterlegte Kategorie und/oder eine Abstandsmessung. Beispielsweise kann auch die Höhe und/oder der Winkel zum Boden und/oder der Winkel wenigstens einer Seitenfläche der Unwegsamkeit bestimmt werden.

Unter dem Begriff Unwegsamkeit wird im Sinne der vorliegenden Erfindung insbesondere wenigstens ein Störobjekt auf oder entlang des Gehweges verstanden, welches bei einem entsprechenden Weitergehen einen Stoß auf das die Prothesen- oder Exoskelettkomponente ausüben kann, sodass die Dämpfereinrichtung aktiv wird.

Grundsätzlich kann es schwierig sein, den Gehweg vorauszusagen, da der Träger oft mehr oder weniger instinktiv einem Hindernis ausweichen wird, wenn es denn nicht über die ganze Breite des Weges ragt. Dann kann es passieren, dass eine Unwegsamkeit erkannt wird und die Stoßdämpfereinrichtung eingestellt oder die Einstellung vorbereitet wird. Anschließend kann durch das Umgehen das Hindernis ausbleiben, sodass das erwartete Ereignis nicht eintritt, weil der Träger den Gehweg ändert. Das stellt in bevorzugten Ausgestaltungen aber kein Problem dar, da die Stoßdämpfereinrichtung in weniger als 30 ms und insbesondere weniger als 10 ms eingestellt werden kann, sodass nicht plötzlich eine Einstellung vorliegt, die dann doch nicht gebraucht wird. Vorteilhafterweise wird die Dämpfereinrichtung mit einem magnetorheologischen Fluid und einem steuerbaren Dämpfungsventil ausgerüstet. Ein Vorteil einer solchen Dämpfereinrichtung ist die enorme Geschwindigkeit, mit der Änderungen eingestellt werden können.

Das bedeutet, dass nicht weit nach vorn "geschaut" werden muss, um eine Unwegsamkeit zu erkennen. Das heißt, dass meist bei Erkennung einer Unwegsamkeit diese auch relevant wird. Vorzugsweise ist ein Erkennen des Umkurvens möglich, wozu vorzugsweise Körper- oder Kopfbewegungen erkannt und berücksichtigt werden.

Die Empfangseinheit ist beispielsweise als eine Kamera ausgebildet und umfasst wenigstens einen Bildsensor. Der Bildsensor ist vorzugsweise dazu geeignet, eine optische Projektion der Unwegsamkeit zu erfassen. Die optische Projektion kann im Bereich des sichtbaren Lichts und/oder im Infrarotbereich und/oder einem Bereich kürzerer oder längerer Wellenlängen erfassbar sein. Die Kamera kann wenigstens ein Objektiv umfassen. Es können wenigstens ein Filter und/oder wenigstens eine Verstärkereinrichtung und/oder Bildstabilisatoreinrichtung vorgesehen sein. Möglich ist auch eine Stereokamera zur Erfassung räumlicher Informationen der Unwegsamkeit.

Vorzugsweise ist die Steuereinrichtung geeignet und ausgebildet ist, die Dämpfung der Dämpfereinrichtung bis zum Erreichen einer erkannten Unwegsamkeit zu verändern und insbesondere härter einzustellen. Das erfolgt insbesondere bei größeren Unwegsamkeiten, deren Höhe z. B. 5 cm oder 10 cm erreicht oder übersteigt. Dadurch kann sichergestellt werden, dass beim Erreichen der Unwegsamkeit noch genügend Federweg zur angemessenen oder optimalen Bewältigung der Unwegsamkeit zur Verfügung steht. Das gilt insbesondere auch für Sprünge und andere vorhersehbare oder absehbare Ereignisse.

Möglich ist es auch, dass die Stoßdämpfereinrichtung wenigstens eine steuerbare Federeinrichtung umfasst. Dabei kann die Erkennungseinrichtung dazu geeignet und ausgebildet sein, in Abhängigkeit der erkannten Unwegsamkeit wenigstens eine Federeigenschaft der Federeinrichtung einzustellen. Zur Einstellung kann z. B. wenigstens ein elektrisch angetriebener Aktor vorgesehen sein.

Die Stoßdämpfereinrichtung kann auch mehrere einzelne oder miteinander vernetzte Dämpfereinrichtungen umfassen. Insbesondere ist wenigstens eine Dämpfereinrichtung der wenigstens einen Dämpfereinrichtung der Stoßdämpfereinrichtung durch die Erkennungseinrichtung steuerbar.

In einer besonders vorteilhaften Ausgestaltung umfasst die Sensoreinheit wenigstens eine Sendeeinheit. Die Sendeeinheit ist vorzugsweise geeignet und ausgebildet, wenigstens ein Signal auszusenden. Die Empfangseinheit ist vorzugsweise dazu geeignet und ausgebildet, wenigstens eine wenigstens teilweise von der Unwegsamkeit ausgehende Reflexion des ausgesendeten Signals zu empfangen und als Signal zu erfassen. Die Erkennungseinrichtung ist vorzugsweise dazu geeignet und ausgebildet, in Abhängigkeit des erfassten Signals die Unwegsamkeit zu erkennen. Die Erkennungseinrichtung ist zudem insbesondere dazu geeignet und ausgebildet, die Dämpfereinrichtung in Abhängigkeit der erkannten Unwegsamkeit zu steuern, sodass wenigstens eine Dämpfungseigenschaft der Dämpfereinrichtung durch ein Signal der Erkennungseinrichtung einstellbar ist. Das reflektierte Signal kann besonders vorteilhaft durch die Erkennungseinrichtung verarbeitet und für eine besonders zuverlässige Erkennung von Unwegsamkeiten herangezogen werden.

Das ausgesendete bzw. empfangene Signal ist insbesondere eine Transversalwelle und/oder eine Longitudinalwelle, beispielsweise eine elektromagnetische Welle und/oder eine Schallwelle. Dabei ist möglich, dass die Welle entsprechenden Modulationen unterzogen wird. Möglich ist auch eine Aussendung als ein Impuls und insbesondere als ein ultrakurzer Impuls. Es kann eine Pulsphasenmodulation vorgesehen sein. Die Unterschiede zwischen den gesendeten und reflektierten Signalen sind beispielsweise charakteristisch für die Größe, die Form und/oder die stoffliche Zusammensetzung der Unwegsamkeit. Die Unterschiede der gesendeten und reflektierten Signale betreffen z. B. die Amplitude, die Frequenz, die Wellenlänge, die Phase und/oder die Polarisation. Es können auch Interferenzen zwischen einem ausgesendeten und einem empfangenen Signal ermittelt werden. Beispielsweise ist die Sensoreinheit als ein Interferometer ausgebildet oder kann ein solches umfassen. Vorzugsweise werden dazu Signale im Bereich kürzerer Wellenlängen eingesetzt, wie z. B. sichtbares Licht.

Vorzugsweise sind die Sendeeinheit und die Empfangseinheit in wenigstens einem gemeinsamen Sensor integriert. Möglich ist es auch, dass die Sendeeinheit und die Empfangseinheit separat ausgebildet sind. Die Übertragung zwischen der Sensoreinheit und der Erkennungseinrichtung kann dabei drahtlos erfolgen. Es kann aber auch wenigstens eine entsprechende Verbindungsleitung vorgesehen sein.

Insbesondere umfasst die Dämpfereinrichtung wenigstens eine erste Dämpferkammer und wenigstens eine zweite Dämpferkammer. Die Dämpferkammern sind insbesondere über wenigstens ein steuerbares Dämpfungsventil miteinander gekoppelt. Die einstellbare Dämpfungseigenschaft ist insbesondere wenigstens ein Maß der Dämpfung. Beispielsweise ist die Dämpfungseigenschaft eine harte oder weiche Dämpfung.

In einer besonders bevorzugten Weiterbildung ist dem Dämpfungsventil wenigstens einer steuerbare Felderzeugungseinrichtung zugeordnet. Die Felderzeugungseinrichtung ist insbesondere dazu geeignet und ausgebildet, durch die Erkennungseinrichtung gesteuert zu werden. Beispielsweise kann dazu eine elektrische Spule vorgesehen sein. Insbesondere ist die Felderzeugungseinrichtung dazu geeignet und ausgebildet, eine Feldstärke in wenigstens einem Dämpfungskanal des Dämpfungsventils zu erzeugen und/oder zu steuern. Dabei ist in dem Dämpfungskanal vorzugsweise ein feldempfindliches rheologisches Medium vorgesehen.

Vorzugsweise ist die Dämpfereinrichtung mit wenigstens einem magnetorheologischen Fluid versehen und weist wenigstens ein einstellbares magnetorheologisches Dämpfungsventil auf. Möglich ist auch wenigstens ein mechanisch ansteuerbares Dämpfungsventil. Besonders bevorzugt wird als rheologisches Medium ein magnetorheologisches Medium und insbesondere ein magnetorheologisches Fluid eingesetzt. Dabei wird insbesondere über die Intensität und Stärke des durch die Felderzeugungseinrichtung aufgebauten Magnetfeldes die resultierende Viskosität des Fluids beeinflusst.

In einer weiteren besonders bevorzugten Weiterbildung ist die Erkennungseinrichtung dazu geeignet und ausgebildet, die Dämpfereinrichtung in Abhängigkeit von Unwegsamkeiten zu steuern, welche in einem Nahbereich liegen. Vorzugsweise werden nur Unwegsamkeiten im Nahbereich berücksichtigt. Insbesondere ist der Nahbereich durch wenigstens eine in der Erkennungseinrichtung hinterlegte Vorgabe definiert, z. B. durch eine Distanz, Länge und/oder Breite und/oder durch einen Winkel. Möglich ist auch ein dynamisch anpassbarer Nahbereich. Der Nahbereich kann auch durch eine Vorgabe des Benutzers einstellbar sein.

Beispielsweise kann der Nahbereich 1m oder 2m oder 3m oder auch 5 m oder auch mehr betragen. Möglich ist auch, dass sich der Nahbereich über 10 m oder 15 m oder sogar 20 m oder mehr erstreckt. Der Nahbereich kann aber besonders bevorzugt auch weniger als 1 m und beispielsweise 70 cm oder 50 cm oder 20 cm oder auch 10 cm oder weniger betragen. Vorzugsweise erstreckt sich der Nahbereich von einem am vorderen Ende der Prothese oder Exoskelett liegenden Bereich in Gehrichtung nach vorne. Beispielsweise beginnt der Nahbereich vor den Zehenspitzen. Besonders bevorzugt ist der Nahbereich kürzer als 5 m und insbesondere kürzer als 2 m.

Die Berücksichtigung von Unwegsamkeiten in einem Nahbereich bietet erhebliche Vorteile, weil beim Gehen und insbesondere beim Gehen im Gelände oft zahlreiche und sehr schnelle Richtungswechsel vorgenommen werden. Eine weit vorausschauende Erkennung wäre somit nicht zweckdienlich und eher nachteilig, da es zu einer Berücksichtigung von Unwegsamkeiten kommen kann, die keine Rolle spielen. Hierbei zeigt sich insbesondere, dass eine Übertragung von aus dem Automobilbereich bekannten Hinderniserkennungssystemen nicht zur Lösung der vorgenannten Aufgabe führt. Im Automobilbereich müssen Hindernisse erkannt werden, welche möglichst weit voraus liegen und eine Gefahr für die Weiterfahrt auf dem gewählten Fahrweg darstellen. Daraus folgend wird dann eine andere Kennlinie des Dämpfers gewählt. Die beim Stand der Technik verwendeten Dämpfer bzw. Luftfahrwerke sind in Relation zum erfindergemäßen Dämpfer "langsam" (Stand der Technik: Es vergehen technologisch bedingt ca. 100ms zwischen dem Erkennen bis zum Erreichen der optimalen Einstellposition des Dämpfers; beim erfindergemäßen Gegenstand sind es < 10ms). Dadurch kann der Stand der Technik viele Aktionen des erfindergemäßen Gegenstandes gar nicht ausführen bzw. durch die schnelle Reaktionszeit des erfindergemäßen Gegenstandes in erfinderischer Kombination mit ausgesuchten Bildverarbeitungs- und Sensorergeben sich ganz neue Möglichkeiten.

Im Prothesenbereich hingegen ist es in der Regel gewünscht, auf die Unwegsamkeiten zuzugehen und diese zu überwinden. Die hier vorgestellte Erfindung soll somit nicht ein Umgehen von Hindernissen, sondern eben gerade ein Über- oder Darübergehen von Unwegsamkeiten durch eine sehr schnelle Anpassung von Dämpfungseigenschaften unterstützen.

Besonders bevorzugt ist die Erkennungseinrichtung auch dazu geeignet und ausgebildet, den Nahbereich in Anhängigkeit der Gehgeschwindigkeit des Prothesen oder Exoskelettträgers vorzugeben. Vorzugsweise erstreckt sich der Nahbereich insbesondere maximal über die Distanz, welche gehend in einer Sekunde zurücklegt. Eine solche Festlegung des Nahbereichs in Abhängigkeit der Gehgeschwindigkeit bietet den Vorteil, dass im Wesentlichen solche Unwegsamkeiten Berücksichtigung finden, welche auch für die Dämpferauslastung relevant sind. Beispielsweise werden solche Unwegsamkeiten, die sich in einer Entfernung von etwa einer Gehsekunde zum Zehenspitze befinden, mit sehr hoher Wahrscheinlichkeit auch übergangen. Möglich ist auch, dass sich der Nahbereich maximal über die Distanz erstreckt welche die Prothese oder das Exoskelett anhand der Gehgeschwindigkeit in weniger als einer Sekunde und beispielsweise in einer halben Sekunde oder besonders bevorzugt in 0,2 Sekunden oder vorzugsweise 0,1 Sekunden oder weniger zurücklegt, was wiederum bei schnellem Gehen im Gelände von großem Vorteil ist. Die erfindergemäße Prothesen- oder Exoskelettkomponente erkennt mittels der Nahfelderkennungssysteme nicht nur Situationen (z. B. eine Wurzel), sondern quantifiziert diese (Abstand bis zur Wurzel, Wurzelhöhe, Wurzellänge, eine oder mehrere Wurzeln ...) und stellt die Prothesen- oder Exoskelettkomponente möglichst spät bis zeitgenau entsprechend ein bzw. regelt diese in Echtzeit aufgrund der Erkennungsinformationen bestmöglich während dem Überwinden des Hindernisses um gleich danach wieder in Echtzeit ein andere Dämpfung einzustellen. Die möglichst späte Umstellung vor und die möglichst rasche Rückstellung (z. B. < 5ms) nach dem Ereignis ist von großer sicherheitstechnischer Bedeutung. Ein wegen z. B. einer Wurzel zu früh oder zu lange auf "ganz weich bzw. nachgiebig" eingestellter Dämpfer erhöht stark die Körperbewegungen und reduziert die Stabilität, instabile Zustände können entstehen und Stürze sind dadurch möglich.

Das Erkennen der Situationen ohne Nahfelderkennungssysteme, mit z. B. mehrachsigen Beschleunigungssensoren, ist beim Stand der Technik ein großes Problem und benötigt viele Sensoren zum Erkennen und Zuordnen von Situationen. Dies erhöht nebst den Kosten auch den Strombedarf. Zudem ist ein enormer Entwicklungsaufwand notwendig, um alle möglichen auftretenden Zustände und Sensorsignale richtig zu analysieren. Eigentlich sind jetzige Systeme ohne Nahfelderkennung in gewisser Weise mit sehbehinderten Menschen zu vergleichen, welche den Alltag erschwert. Ein Blindenstock, ein Blindenhund und insbesondere das "Augenlicht" hat also einen unbestrittenen und nachweislich großer Vorteil.

Wenn z. B. die Prothese eine Situation (Stiege bzw. Stufen abwärts) nicht richtig erkennt und der Prothesenträger dann hinfällt, weil die Dämpfung zu weich oder falsch eingestellt wird, führt dies nebst den gegebenenfalls schweren Verletzungen zum Vertrauensverlust in die Prothese. Dies beeinträchtigt das Trageverhalten wesentlich.

Die bestens nicht Nahfelderkenungssensoren in/an der Prothese können z. B. einen quer über den Waldweg stehenden Ast oder ein Seil etc. nicht erkennen. Der schnell laufende Prothesenträger kann hier stolpern, weil der Dämpfer das Bein beim Darübergehen-/springen nicht länger als zuvor gebeugt lässt, so wie es der Mensch instinktiv macht, wenn er das Hindernis sieht und nicht mehr ausweichen kann und darüber springt. Sieht und identifiziert das Umfelderkennungssystem das Hindernis, so kann die optimale Abwinklung der Prothese errechnet und entsprechend angesteuert werden. Das Gesamtpaket ist dann situationsabhängig und vorausschauend, der Prothesenträger verletzt sich nicht.

Stiegen oder Stufen oder Rampen stellen z. B. hohe Anforderungen an die Dämpfer, da die Kräfte hierbei viel höher wie z. B. beim ebenen Gehen sind: Ebenes Gehen benötigt ca. 1500N Dämpfkraft. Eine Treppe oder eine Rampe abwärts benötigen hingegen bis 6.000N. Somit muss der Dämpfer schon beim (oder vor dem) ersten Abwärtstritt auf viel härter (bis Faktor 3) geschaltet sein (werden), sonst verfährt der Dämpfer aufgrund der weichen Einstellung und den hohen eingebrachten Kräfte zu schnell und zu viel Weg wird verschenkt, bis die ideale Dämpfung erkannt und eingestellt ist (Stand der Technik: ca. 60ms Ventilverstellzeit). Mitunter kann es jetzt sogar vorkommen, dass bis der Dämpfer richtig eingestellt (verstellt) ist, dieser schon den gesamten Verfahrweg gemacht hat und im Endanschlag ist. Somit sackt der Prothesenträger viel zu schnell ein, um hernach abrupt in den Anschlag zu kommen, was zum Sturz oder starken Belastungen führen kann. Durch frühzeitige Erkennung dieser Situation mittels der hier beschriebenen Sensoren kann dies erkannt und vermieden werden.

Ein anderes Beispiel ist z. B., wenn ein Sprung erkannt wird (z. B. zwei Stiegen werden übersprungen ...) und die ungefähre Landezeit/-punkt errechnet wird. Das Gesamtsystem steuert dann Dämpfer entsprechend bestmöglich = harte Dämpfung, damit der Prothesenträger nicht zu stark in die Knie geht und evtl. dadurch Vorderlage des Oberkörpers bekommt mit anschließendem Sturz.

Möglich ist es aber auch, dass sich der Nahbereich über die Distanz erstreckt, welche das Prothesen oder Exoskelett anhand der Gehgeschwindigkeit in mehr als einer Sekunde und beispielsweise 1,5 Sekunden oder 2 Sekunden oder 3 Sekunden zurücklegt. Zur Bestimmung der Gehgeschwindigkeit kann die Prothesen- oder Exoskelettkomponente geeignete Sensoren umfassen. Es kann auch ein Tachometer und/oder Navigationssystem des Prothesen- oder Exoskelettträgers abgefragt werden.

Die Zeit, mittels der die Erstreckung des Nahbereichs definiert wird, kann in Abhängigkeit einer Breite des Nahbereichs festgelegt werden. Die Breite des Nahbereichs erstreckt sich dabei insbesondere quer zur Gehrichtung. Die Breite des Nahbereichs kann auch durch einen Erfassungswinkel der Sensoreinheit festgelegt werden. Der Erfassungswinkel ist beispielsweise dadurch bestimmt, in welchem Winkel das Signal ausgesendet und/oder in welchem Winkel die Empfangseinheit ein reflektiertes Signal empfängt. Eine solche Ausgestaltung ist vorteilhaft, da die Breite des Nahbereichs einen Einfluss darauf hat, wie schnell eine erkannte Unwegsamkeit durch ein Ausweichmanöver umgangen werden kann und somit für die Dämpfersteuerung irrelevant wird.

Die Erkennungseinrichtung ist bevorzugt dazu geeignet und ausgebildet, in Folge einer erkannten Unwegsamkeit im Nahbereich die Dämpfungseigenschaft der Dämpfereinrichtung in weniger als 30 Millisekunden einzustellen. Unter diesem Zeitraum ist dabei insbesondere die Zeit zu verstehen, die nach einer Erkennung einer Unwegsamkeit für die entsprechende Dämpfereinstellung benötigt wird. Vorzugsweise erfolgt auch die Auswertung der Sensorsignale und die Erkennung der Unwegsamkeit in einem solchen und besonders bevorzugt deutlich kürzeren Zeitraum.

Insbesondere ist auch die Dämpfereinrichtung dazu geeignet und ausgebildet, in der genannten Zeit von der Erkennungseinrichtung eingestellt zu werden. Eine solche kurze Einstellungszeit hat den Vorteil, das entsprechend kurze Nahbereiche verwirklicht werden können. Das erhöht die Wahrscheinlichkeit, dass die erkannten Unwegsamkeiten auch wirklich relevant werden. Ein weiterer Vorteil ist, dass bei einem plötzlichen Manöver und einer somit notwendig gewordenen Neuerkennung von Unwegsamkeiten immer noch eine vollständige Anpassung der Dämpfungseigenschaft möglich ist.

Besonders bevorzugt erfolgt die schnelle Einstellung der Dämpfungseigenschaft mittels des Dämpferventils und der zugeordneten Felderzeugungseinrichtung. Insbesondere erfolgt die Einstellung der Dämpfungseigenschaft der Dämpfereinrichtung in weniger als 20 Millisekunden oder weniger als 10 Millisekunden und besonders bevorzugt in weniger als 5 Millisekunden. Die Einstellung kann auch in weniger als 3 Millisekunden und insbesondere in weniger als 2 Millisekunde erfolgen. Möglich ist aber auch eine Einstellung, welche länger als 30 Millisekunden und beispielsweise 50 Millisekunden beträgt.

Die Erkennungseinrichtung ist insbesondere dazu geeignet und ausgebildet, die Höhe der Unwegsamkeit über dem Boden zu ermitteln und für die Steuerung der Dämpfereinrichtung zu berücksichtigen. Vorzugsweise ist die Erkennungseinrichtung dazu geeignet und ausgebildet den Winkel wenigstens eines Bereichs der Unwegsamkeit zum Boden zu ermitteln und für die Steuerung der Dämpfereinrichtung zu berücksichtigen. Beispielsweise kann die Dämpfung desto weicher eingestellt werden, je steiler und/oder höher die Unwegsamkeit ist.

Die Steuerung erfolgt dabei beispielsweise im Sinne einer Kennfeldsteuerung, sodass entsprechende Werte von Höhe und/oder Winkel hinterlegten Werten für die Dämpferhärte zugeordnet werden. Möglich ist auch eine schwellenwertabhängige Steuerung, sodass bei einem Über- und/oder Unterschreiten von Schwellenwerten bzgl. Höhe und/oder Winkel eine zugeordnete Dämpferhärte eingestellt wird. Möglich ist auch, dass die Steuerung lernfähig ausgebildet ist. Dazu kann die Steuerung beispielsweise einen lernfähigen Algorithmus und/oder eine Fuzzylogik und/oder einen Algorithmus im Prinzip eines neuronalen Netzwerkes oder dergleichen umfassen.

In einer vorteilhaften Ausgestaltung ist die Erkennungseinrichtung dazu geeignet und ausgebildet, eine Distanz zu der Unwegsamkeit zu ermitteln. Dabei kann es sein, dass die von der Sensoreinheit erfassten Signale charakteristisch für einen Abstand der Unwegsamkeit zu der Sensoreinheit sind. Beispielsweise kann ein Korrekturfaktor vorgesehen sein, mittels welchem ein aus dem Abstand zwischen Sensoreinheit und Unwegsamkeit der Abstand zwischen dem Bein und der Unwegsamkeit berechenbar ist.

Anhand der Information über den Abstand zwischen Zehenspitze und Unwegsamkeit kann der Zeitpunkt berechnet werden, wann der Fuß mit der Unwegsamkeit in Kontakt tritt. So kann beispielsweise unter Berücksichtigung der Gehgeschwindigkeit die Dämpfereinstellung genau dann vorgenommen werden, wenn die Anpassung an die Unwegsamkeit erforderlich ist - und zwar sogar just in dem Moment, in welchem es benötigt wird. In Kombination mit einer zuvor beschriebenen Ausgestaltung, bei welcher eine Felderzeugungseinrichtung zur Dämpfereinstellung verwendet wird, sind dann sehr schnelle und kurzfristige Reaktionen möglich, sodass auch auf direkt vor dem Fuß liegende Unwegsamkeiten noch mit einer optimalen Dämpfereinstellung reagiert werden kann.

Insbesondere kann bei vorausschauender Erkennung einer Unwegsamkeit wie ein auf der Fahrbahn oder Gehweg liegender Gegenstand, einer dicken Wurzel oder dergleichen Federweg vor der Unwegsamkeit eingespart werden, um für die Überwindung der Unwegsamkeit (Wurzel, Stein etc.) sicher noch genug Federweg zur optimalen Dämpfung zur Verfügung zu haben. Ohne vorausschauende Erkennung könnte es erforderlich werden, die Dämpfung beim Übergehen der Unwegsamkeit viel härter einzustellen als eigentlich gewünscht wäre, wenn denn der zur Verfügung stehende Federweg schon weitgehend aufgebraucht ist oder ein Durchschlag droht.

In allen Fällen ist es auch möglich, dass bereits aus der Erkennung einer Unwegsamkeit an sich auf dessen Entfernung zurückgeschlossen werden kann. Dann ist eine zusätzliche Entfernungsbestimmung vorzugsweise nicht notwendig. Das ist beispielsweise dann der Fall, wenn der Erfassungsbereich der Sensoreinheit entsprechend fokussiert ist, sodass ohnehin nur in einer bestimmten Entfernung liegende Unwegsamkeiten registriert werden. Dann kann z. B. direkt nach einer Erkennung der Unwegsamkeit unter der Berücksichtigung der Gehgeschwindigkeit und eines Entfernungsfaktors der Zeitpunkt ermittelt werden, wann die Prothese mit der Unwegsamkeit in Kontakt tritt.

Die Erkennungsweinrichtung ist vorzugsweise auch dazu geeignet und ausgebildet, bei der Steuerung der Dämpfereinrichtung wenigstens einen voreingestellten Grenzwert für eine maximale Dämpfung zu berücksichtigen. Die Steuerung kann auch einen Grenzwert für eine minimale Dämpfung berücksichtigen. Der Grenzwert kann dabei vom Benutzer festlegbar sein. Möglich sind auch Grenzwerte in Form von Werksweinstellungen. Möglich ist aber auch, dass der Grenzwert nach wenigstens einer Benutzereingabe automatisch festgelegt wird. Beispielsweise kann der Fahrer sein Gewicht mittels einer Benutzerschnittstelle eingeben und anschließend wird ein Grenzwert für die minimale bzw. maximale Dämpfung festgelegt. Gegebenenfalls wird das Gewicht des Trägers sensorisch ermittelt.

Solche Ausgestaltungen haben den Vorteil, dass die Einstellung der Dämpfereinrichtung durch die Erkennungseinrichtung nicht zu Einstellungen führt, welche problematisch oder vom Benutzer unerwünscht sind. Es kann auch vorgesehen sein, dass bei bestimmten Situationen, in denen eine hohe Dämpferauslastung sensiert wird, die Steuerung in Abhängigkeit erkannter Unwegsamkeiten kurzfristig deaktiviert wird. Dazu kann z. B. eine Dämpfersteuerung vorgesehen und ausgebildet sein, welche Dämpfersensoren umfasst und die Steuerbefehle der Erkennungseinrichtung nach einer bestimmten Priorität ausführt.

In einer vorteilhaften Weiterbildung ist die Prothesen- oder Exoskelettkomponente für eine Stoßdämpfereinrichtung mit wenigstens einer ersten und wenigstens einer zweiten Dämpfereinrichtung vorgesehen. Dabei ist die erste Dämpfereinrichtung vorzugsweise ein Hüftdämpfer und die zweite Dämpfereinrichtung vorzugsweise einem Kniedämpfer zugeordnet. Die Erkennungseinrichtung kann insbesondere dazu geeignet und ausgebildet sein, die zweite Dämpfereinrichtung zeitverzögert zu der ersten Dämpfereinrichtung einzustellen. Die Erkennungseinrichtung ist vorzugsweise dazu ausgebildet, die Dämpfereinrichtungen unabhängig voneinander einzustellen.

Eine derartige zeitverzögerte Ansteuerung kann den Vorteil haben, dass auch die Dämpfereinrichtung des Knies optimal vorbereitet und zum optimalen Zeitpunkt auf die Unwegsamkeit angepasst werden kann. Dabei ist bevorzugt, dass die Erkennungseinrichtung dazu geeignet und ausgebildet ist, die Zeitverzögerung in Abhängigkeit der Gehgeschwindigkeit des Prothesen- oder Exoskelettträgers anzupassen. Insbesondere wird dabei auch die Kinematik zwischen Hüfte und Knie berücksichtigt. Es ist möglich, dass der Dämpfereinrichtung wenigstens eine Detektoreinrichtung bzw. ein Sensormodul zur Erfassung einer Dämpferauslastung zugeordnet ist. Beispielsweise kann eine Detektoreinrichtung (Dämpfersensor) vorgesehen sein, welche den Weg und/oder die Geschwindigkeit zweier zueinander beweglichen Bauteile der Dämpfereinrichtung erfasst. Insbesondere kann mittels der Detektoreinrichtung erfasst werden, wie weit und/oder wie schnell der Dämpfer bei einem Stoß zusammenfährt und/oder nach einem Stoß wieder auseinander fährt. Die Detektoreinrichtung kann dabei ein Teil der Prothese oder des Exoskeletts sein, welches der Prothesen- oder Exoskelettkomponente zugeordnet ist. Möglich ist aber auch, dass die Detektoreinrichtung von der Prothesen- oder Exoskelettkomponente umfasst ist.

Beispielsweise sind einige Dämpfersteuerungen bereits werksseitig mit entsprechenden Sensoren zur Erkennung der Auslastung ausgerüstet, insbesondere um eine selbstständige Anpassung vornehmen zu können. Bevorzugt ist die Erkennungseinrichtung dazu geeignet und ausgebildet, derartige Sensordaten auszulesen und bei der Einstellung der Dämpfereigenschaft zu berücksichtigen.

Bevorzugt ist die Erkennungseinrichtung dazu geeignet und ausgebildet, die Dämpferauslastung nach einem Stoß einer zuvor erkannten Unwegsamkeit zu registrieren. Das ermöglicht einen Rückschluss, ob die als Reaktion auf die erkannte Unwegsamkeit erfolgte Einstellung der Dämpfereigenschaft zielführend war oder nicht. Bevorzugt kann die Erkennungseinrichtung die registrierte Dämpferauslastung mit in wenigstens einer Speichereinrichtung hinterlegten Werten für eine Dämpferauslastung vergleichen. Besonders bevorzugt ist die Erkennungseinrichtung so ausgebildet, dass die Steuerung der Dämpfereinrichtung angepasst werden kann, wenn die registrierte Dämpferauslastung von wenigstens einem vorgegebenen Maß für die Dämpferauslastung abweicht. Dabei wird die Anpassung der Steuerung vorzugsweise so vorgenommen, dass bei zukünftigen Unwegsamkeiten eine bessere Dämpferauslastung im Bereich des vorgegebenen Maßes erreichbar ist. Vorzugsweise ist die Erkennungseinrichtung mit wenigstens einem lernfähigen Algorithmus ausgestattet.

Insbesondere ist die Erkennungseinrichtung so ausgebildet, dass sie die vorgenommenen Dämpfereinstellungen selbstständig kontrollieren kann und bei einem Auftreten von ungünstigen Dämpferauslastungen zukünftige Steuerbefehle an die Dämpfereinrichtung um wenigstens einen Korrekturfaktor anpasst, sodass die Dämpferauslastung zukünftig wieder in einem optimalen Bereich liegt.

Es kann auch wenigstens ein Sensormodul zur Erfassung einer Federauslastung wenigstens einer Federeinrichtung vorgesehen sein. Dabei ist die Erkennungseinrichtung insbesondere dazu geeignet und ausgebildet, das Sensormodul auszulesen und die Steuerung der Federeinrichtung anzupassen, wie es zuvor auch für die Dämpferauslastung beschrieben wurde.

Es ist bevorzugt, dass die Erkennungseinrichtung wenigstens eine Speichereinrichtung zur Ablegung der erkannten Unwegsamkeiten aufweist. Die Speichereinrichtung ist vorzugsweise mit wenigstens einer Schnittstelle wirkverbunden, sodass ein Auslesen der aufgezeichneten Unwegsamkeiten möglich ist, beispielsweise durch einen Benutzer. Möglich ist auch, dass die Erkennungseinrichtung die Speichereinrichtung selbstständig auslesen kann, beispielsweise für eine Selbstkorrektur. Möglich ist auch, dass die Speichereinrichtung zur Aufzeichnung der Dämpferauslastung und/oder der vorgenommenen Dämpfereinstellungen ausgebildet ist. Die Erfassung solcher Daten ermöglicht dem Benutzer eine besonders einfache Kontrolle seiner Einstellungen an der Stoßdämpfereinrichtung.

Es ist besonders bevorzugt, dass die Sensoreinheit an wenigstens einem Bauteil der Prothese oder des Exoskeletts angeordnet ist.. Das hat den Vorteil, dass die Sensoreinheit im Wesentlichen stets in die Gehrichtung ausgerichtet ist. Möglich ist auch die Anordnung der Sensoreinheit an einem anderen Körperteil des Trägers. Dabei ist möglich, dass die Sendeeinheit und die Empfangseinheit beabstandet und/oder an separaten Komponenten angeordnet sind. Vorzugsweise ist die Sensoreinheit verschwenkbar an wenigstens einer Halteeinrichtung aufgenommen. Dabei ist insbesondere die Haltereinrichtung mit einem Befestigungselement ausgebildet, welches an einer Komponente der Prothese oder Exoskeletts angeordnet wird. Zudem umfasst die Haltereinrichtung wenigstens ein zweites Befestigungselement, welches zur Verbindung mit der Sensoreinheit vorgesehen ist. Vorzugsweise ist eine werkzeuglose Montage bzw. Demontage der Sensoreinheit an der Haltereinrichtung vorgesehen. Insbesondere kann auch die Halteeinrichtung werkzeuglos an der Prothesen oder Exoskelett befestigt bzw. demontiert werden.

Besonders bevorzugt ist die Sensoreinheit mittels der Haltereinrichtung verschwenkbar an der Prothese oder dem Exoskelett montiert. Die Verschwenkbarkeit ist insbesondere so ausgebildet, dass der Sendewinkel und/oder der Empfangswinkel der Sensoreinheit zum Boden einstellbar ist. Beispielsweise kann eine Skalierung und/oder eine Rasterung an der Haltereinrichtung vorgesehen sein, sodass dem Benutzer eine Hilfestellung bei der Ausrichtung der Sensoreinheit gegeben wird. Ein derartiges Verschwenken der Sensoreinheit hat den Vorteil, dass der Erfassungsbereich für die Erkennung von Unwegsamkeiten schnell und unaufwendig angepasst werden kann.

Insbesondere erfolgt nach einer derartigen Verschwenkung der Sensoreinheit eine Anpassung der Erkennungseinrichtung hinsichtlich der Dämpferansteuerung nach erkannten Unwegsamkeiten. Besonders einfach ist eine derartige Anpassung dann, wenn die Erkennungseinrichtung lernfähig ausgebildet ist. Dann kann die Erkennungseinrichtung beispielsweise nach dem Überfahren einer oder mehrerer Unwegsamkeiten unter Berücksichtigung der Gehgeschwindigkeit selbstständig den Abstand zwischen dem Erfassungsbereich der Sensoreinheit und dem Benutzer ermitteln und danach die Dämpferansteuerung justieren.

Möglich ist aber auch, dass ein Verschenken der Sensoreinheit und/oder eine sonstige Veränderung des Erfassungsbereichs der Sensoreinheit eine manuelle Einstellung an der Erkennungseinrichtung erfordert. Beispielsweise kann an der Sensoreinheit eine Lichtquelle angeordnet sein, welche einen Leuchtpunkt in den Erfassungsbereich sendet. Dann kann der Benutzer den Abstand zwischen dem Leuchtpunkt und dem Fuß ausmessen und den gemessenen Abstand im Sinne eines Korrekturfaktors über eine Eingabeeinrichtung oder sonstige Schnittstelle in die Erkennungseinrichtung eingeben.

Insbesondere ist die Sensoreinheit an wenigstens einer Haltereinrichtung angeordnet, sodass sich eine beabstandete Anordnung zu wenigstens einer Komponente der Prothese oder des Exoskeletts ergibt. Beispielsweise kann ein Bügel vorgesehen sein. In einer weiteren bevorzugten Ausgestaltung ist die Sensoreinheit als ein Ultraschallsensor ausgebildet oder umfasst wenigstens einen solchen. Derartige Ultraschallsensoren sind kostengünstig und weisen sehr kompakte Abmessungen auf. Zudem ist mit solchen Sensoren eine Ausgestaltung des Erkennungssystems mit sehr geringem Gewicht möglich, was besonders im Sportradbereich ein wichtiges Merkmal ist. Zudem ist mit Ultraschallsensoren eine zuverlässige Erkennung von Unwegsamkeiten und insbesondere von deren Höhe, Winkel und/oder Abstand möglich. Möglich ist auch, dass die Sensoreinheit zwei oder drei oder mehrere Ultraschallsensoren umfasst. So kann beispielsweise ein 2-, 4- und/oder 6-Kanal und/oder Mehr-Kanalsystem vorgesehen sein.

Die Sensoreinheit kann auch wenigstens einen Infrarotsensor umfassen oder als ein solcher ausgebildet sein. Auch Infrarotsensoren bieten eine kostengünstige und zuverlässige Sensorik zur Erkennung von Unwegsamkeiten und deren Geometrie bzw. Abstand. Dabei können auch zwei oder drei oder mehr Infrarotsensoren vorgesehen sein.

Kostengünstige Tiefensensoren, wie z. B. in der Microsoft Kinect verwendet, können auch eingesetzt werden.

Die Sensoreinheit kann auch ein Head Mounted Displays (HMD), ein near-to-eye optical systems (wie z. B. Google Glas) oder eine Weiterentwicklung dieser Technologie sein, oder durch diese ergänzt werden. Damit erhält man u.a. auch eine Zusatzinformation, weil erkannt wird: In welche Richtung und wohin bzw. worauf schaue ich (Drehwinkel des Kopfes, Neigung des Kopfes, Fokus; Bewegungsgeschwindigkeit ...). Der Träger des HMD schaut hier automatisch auf die für die Situation relevanten Dinge und macht so eine Vorselektion. Dies erleichtert die Situationsanalyse und die Treffsicherheit der Nahfelderkennung. Möglich ist es auch dem Träger eine Information einzublenden (z. B. Geschwindigkeit verringern) um so in Kombination mit der aus dem Sensorsignal abgeleiteten Anpassung der Dämpfer ein besseres Ergebnis zu erzielen.

Möglich ist auch, dass die Sensoreinheit als wenigstens ein Radarsensor ausgebildet ist oder wenigstens einen solchen umfasst. Beispielsweise kann die Sensoreinheit als ein sog. Ultrabreitbandradarsensor ausgebildet sein. Dabei ist die Sensoreinheit dazu ausgebildet, wenigstens einen ultrakurzen Impuls auszusenden und die entsprechenden Reflektionen wieder zu empfangen und auszuwerten. Beispielsweise kann eine Veränderung der Phase, Frequenz, Wellenlänge und/oder Laufzeit zur Erkennung der Unwegsamkeit herangezogen werden.

Eine Prothesen- oder Exoskelettkomponente kann auch weitere Komponenten und Teile umfassen, sodass eine Prothesen- oder Exoskelettkomponente auch eine vollständige Prothese oder ein vollständiges Exoskelett bilden kann.

Das erfindungsgemäße Verfahren eignet sich zum Betreiben einer Prothesen- oder Exoskelettkomponente, welche für eine Prothese oder Exoskelett vorgesehen ist. Die Prothese oder das Exoskelett umfasst wenigstens eine Stoßdämpfereinrichtung mit wenigstens einer Dämpfereinrichtung. Die Dämpfereinrichtung ist über wenigstens eine Steuereinrichtung steuerbar. Dabei wird mittels einer Erkennungseinrichtung wenigstens ein Signal und insbesondere ein von wenigstens einer Unwegsamkeit beeinflusstes oder ausgehendes Signal berührungslos erfasst. Anhand des berührungslos erfassten Signals wird die Unwegsamkeit erkannt. In Abhängigkeit der erkannten Unwegsamkeit wird die Dämpfereinrichtung gesteuert. Dabei wird wenigstens eine Dämpfungseigenschaft der Dämpfereinrichtung eingestellt.

Das erfindungsgemäße Verfahren hat den Vorteil, dass Unwegsamkeiten automatisch erkannt und die Stoßdämpfer daraufhin so eingestellt werden, dass die Unwegsamkeiten mit möglichst optimalen Dämpfungseigenschaften passiert werden.

Erfindungsgemäß wird mittels der Erkennungseinrichtung wenigstens ein Signal ausgesendet und wenigstens eine von der Unwegsamkeit ausgehende Reflexion des ausgesendeten Signals empfangen und als Signal erfasst.

Nach Erkennung der Unwegsamkeit bis zum Erreichen der Unwegsamkeit wird die Dämpfereinrichtung vorzugsweise härter eingestellt. Insbesondere kann durch die vorausschauende Erkennung einer Unwegsamkeit genügend Federweg für die Unwegsamkeit eingespart werden, sodass eine optimale Dämpfung der Unwegsamkeit erfolgen kann.

Besonders bevorzugt kommt in dem erfindungsgemäßen Verfahren eine Prothesen- oder Exoskelettkomponente zum Einsatz, wie sie in einem der vorhergehenden Ansprüche beschrieben wurde. Besonders bevorzugt ist auch, dass die Dämpfereinrichtung anhand einer steuerbaren Felderzeugungseinrichtung und mittels eines feldempfindlichen rheologischen Mediums eingestellt wird.

Bevorzugt werden dem erfindungsgemäßen Verfahren nicht nur dynamische Hindernisse, Gehwegverläufe, Geländeformen usw. erfasst und entsprechend steuerungstechnisch berücksichtigt, sondern auch statische Hindernisse oder Situationen:
Beim Aufenthalt auf einem Empfang oder in einer Bar (langes ruhiges Stehen) kann so steuerungstechnisch (Abtastrate, Strombedarf, erste Einstellung ...) anders behandelt werden wie das Warten vor einem Lift oder bei einer roten Ampel (kurzes Stehen mit nachfolgendem Tritt/Gehsteigkante und mitunter schnellem Loslaufen ...) .

Steht der Prothesenträger an der Theke mit durchgestrecktem Knie, wird keine Haltekraft benötigt. Hier kann dann der Aktor, bevorzugt ein Aktor mit magnetorheologische Flüssigkeit, die Abfragezeit (Takt der Elektronik) um Strom zu sparen reduziert werden. Dies geht bis zu einem stark Strom sparenden "Sleepmodus". Wird die Situation nicht erkannt und der MRF Aktor stromlos geschaltet, kann dies problematisch bzw. gefährlich werden, wenn der Prothesenträger sich ganz plötzlich bewegt und das Ventil ganz offen ist (weil stromlos) und deshalb einknickt. Um hier entgegen zu wirken, müssten also trotz des ruhigen Stehens = kein Strombedarf für das MRF-Ventil, kurze interne Elektronikabfragezeiten eingestellt werden, was den Strombedarf erhöht. Dies auch deshalb, weil z. B. das Stehen an einer Bar ein Vorgang sein kann, der über Stunden geht. Die Reduktion des Strombedarfs ist sehr wichtig, weil dadurch Gewicht, Bauraum gespart und die Nutzungsdauer erhöht wird.

Mittels zumindest einem Sensor wie z. B. einem Head up Display mit Bild- und/oder Spracherkennung (fortgeschrittene Datenbrille) kann erkannt werden, ob der Prothesenträger sich wegbewegen will oder nicht. Er wird zuerst die Umgebung entsprechend erkannt (Drehen des Kopfes; evtl. über Spracherkennung und die Analyse des Gesprochenen wie "Tschüss"; "ich gehe jetzt"; "Zahlen der Rechnung bitte") und dann loslaufen. In Kombination mit der Umgebungserkennung und Situationsanalyse kann die Situation besser und manchmal überhaupt nur bewertet werden.

Nach dem Stand der Technik benötigt man Arm/Finger/Gelenkstummel... zum Steuern einer z. B. Prothese oder auch einer aktiven Prothese. Dies funktioniert bei vielen Personen nicht, weil diese z. B. schwer behindert sind (z. B. ab der Halswirbelsäule gelähmt). Sensoren und eine Spracherkennung und Bilderkennung unterstützen Geh- und andere Hilfen. Diese lassen sich dadurch besser steuern. Die Sensoren können hier helfen, indem Situationen erkannt und daraus resultierend Aktionen ausgeführt werden, wie zuvor beschrieben. Vorteilhaft sind diese in Kombination mit Sprachbefehlen.

Am Anfang der Entwicklung gibt es ein autonomes System (z. B. Prothesen), dessen Verhalten durch das "Vorausschauen" verbessert wird. Nach und nach, mit zunehmender Verfeinerung der Sensoren usw. übernimmt das "Vorausschauen" mehr Funktionen.

Die erfindergemäßen Prothesen oder Exoskelettkomponenten können von Prothesenträgern auch beim Skifahren, Surfen oder anderen Anwendungen eingesetzt werden.

Das "Vorausschauen" muss nicht unbedingt optisch sein, sondern kann auch akustisch sein. Über einen akustischen Sensor (Mikrofon; Frequenz, Lautstärke, Spracherkennung ...) kann die Oberfläche beim schnellen Gehen (Teer = leise; Schotter = laut; Windgeräusche = schnell ...) benutzt und daraus resultierend die Dämpfereinstellung verändert werden. Dies gilt auch für akustische Hinweise und Warnungen von Mitmenschen wie auch automatische Hinweise und Warnungen oder Informationen (Lifttür schließt, Achtung Rolltreppe ...).

Typischerweise müssen auch Prothesen- oder Exoskelettbenutzer wenigstens gelegentlich auch Lasten wie einen Rucksack tragen oder Gegenstände anheben und tragen, ein Paket entgegennehmen etc. Die Sensor- bzw. die Bilderkennungseinheit samt Auswertung erkennt hier vorzugsweise vorausschauend schon die Situation bzw. schätzt z. B. die Last, den Lastabstand und den Winkel ab. Es können auch ausgestreckte Arme und abgewinkelte Arme etc. berücksichtigt werden. Die Steuereinrichtung passt den Dämpfer entsprechend an, z. B. wird der oder es werden die Dämpfer härter eingestellt und/oder die Kennlinien angepasst. So kann z. B. ein zu rasches Verfahren in den Endanschlag oder andere ungünstige Situationen vermieden werden.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine schematische Ansicht eines mit einer erfindungsgemäßen Prothesen- oder Exoskelettkomponente ausgerüstete Prothese;
- Fig. 2: eine schematische Ansicht der Steuerungsstruktur des Prothese oder des Exoskeletts nach Fig. 1;
- Fig. 2a: eine schematische Ansicht der Prothese nach Fig. 1 in einem Gelände; und
- Fig. 3: eine schematische Ansicht einer Stoßdämpfereinrichtung für die Prothese nach Fig. 1.

Mit Bezug auf die beiliegenden Figuren wird im Folgenden ein Ausführungsbeispiel einer bzw. eines mit einer Prothesen- oder Exoskelettkomponente 401 und mit Stoßdämpfern 100 ausgerüsteten Prothese oder Exoskelett 200 erläutert.

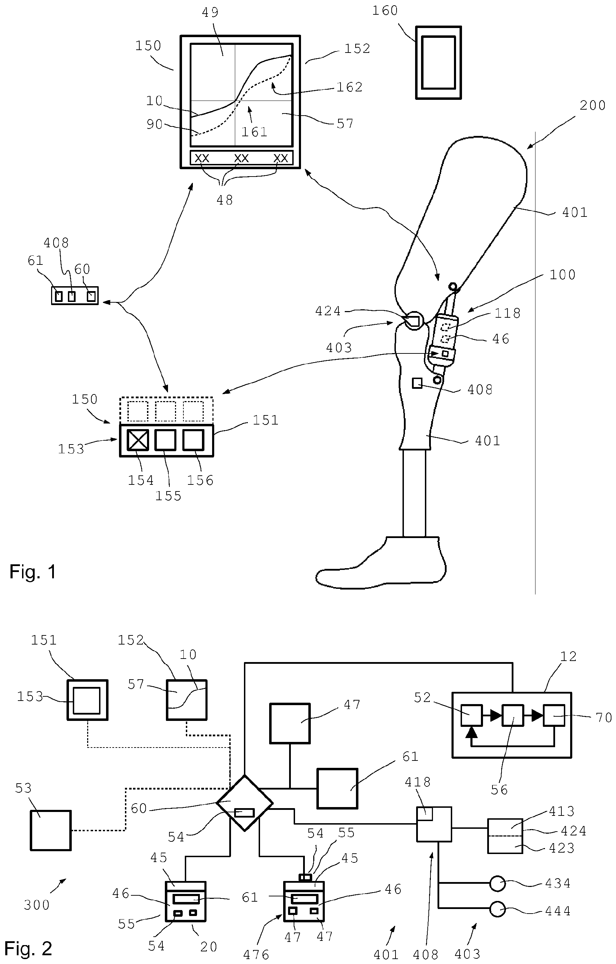

Fig. 1 zeigt eine schematische Darstellung einer Prothese 200. Die Prothese verfügt über einen Stoßdämpfer 100, der wenigstens eine Dämpfereinrichtung umfasst. Eine zentrale Steuereinrichtung 60 ist hier zusammen mit einer Batterieeinheit 61 in einem Behälter vorgesehen.

Zusätzlich weist hier jeder Stoßdämpfer 100 wenigstens eine Steuereinrichtung 46 an einer austauschbar vorgesehenen Elektronikeinheit auf. Die Elektronikeinheiten können jeweils separate Batterieeinheiten aufweisen. Bevorzugt ist aber eine Energieversorgung durch die zentrale Batterieeinheit 61.

Die Dämpfersteuerung 200 und die zentrale Steuereinrichtung 60 werden über Bedieneinrichtungen 150 bedient. Es sind zwei Bedieneinrichtungen 150 vorgesehen, nämlich eine Betätigungseinrichtung 151 und eine Einstelleinrichtung 152. Die Betätigungseinrichtung 151 weist mechanische Eingabeeinheiten 153 auf. Die Einstelleinrichtung 152 kann als Computer ausgeführt sein. Möglich ist es aber auch, dass ein Smartphone 160, eine Smartwatch (Smartdevice) oder ein Tablett oder dergleichen als Einstelleinrichtung 152 eingesetzt wird und beispielsweise in der Tasche oder in dem Rucksack des Benutzers aufbewahrt wird, wenn keine Veränderung der Einstellungen vorgenommen wird.

Möglich ist es auch, dass zwei Stoßdämpfer mit einer Betätigungseinrichtung 151 synchron gesteuert werden.

Das Display 49 ist insbesondere als grafische Bedieneinheit oder Touchscreen 57 ausgeführt, sodass der Benutzer beispielsweise eine dargestellte Dämpferkennlinie 10 mit den Fingern berühren und durch Ziehen ändern kann. Dadurch kann aus der durchgezogen dargestellten Dämpferkennlinie 10 durch Berühren an einem oder mehreren Punkten die dargestellte Dämpferkennlinie 90 erzeugt werden, die dann ab sofort für die Steuerung eingesetzt wird. Die Veränderung der Dämpferkennlinien 10, 90 ist auch im Betrieb zum Beispiel beim Gehen. Hier wird nicht nur die Dämpfung verändert, sondern es kann auch gleichzeitig oder aber auch nur die Federung verändert werden.

Die Einstelleinrichtung 152 kann auch als Displaycomputer dienen und Informationen über die aktuelle Geschwindigkeit, sowie über die Durchschnittsgeschwindigkeit und/oder die Tage-, Tour, Runden- und Gesamtkilometer anzeigen. Möglich ist auch die Anzeige der aktuellen Position, der aktuellen Zeit, der momentanen Höhe, der gegangenen Strecke sowie des Streckenprofils und auch eine mögliche Reichweite unter den aktuellen Dämpfungsbedingungen.

Die hier gezeigte Prothese 200 ist mit einer erfindungsgemäßen Prothesen- oder Exoskelettkomponente 401 ausgestattet. Die gezeigte Prothese 200 mit mit der Prothesen- oder Exoskelettkomponente 401 nach dem erfindungsgemäßen Verfahren gesteuert werden kann.

Die Prothesen- oder Exoskelettkomponente 401 umfasst in der hier gezeigten Ausgestaltung eine Erkennungseinrichtung 408, welche in die zentrale Steuereinrichtung 60 integriert ist. Die Erkennungseinrichtung 408 kann aber auch separat ausgebildet und an einem beliebigen geeigneten Ort an dem Prothesenträger 200 untergebracht sein. Die Prothesen- oder Exoskelettkomponente 401 umfasst hier zudem eine Sensoreinheit 403, welche einen an dem Prothesenträger angebrachten Ultraschallsensor 424 umfasst. Die Sensoreinheit 403 ist hier über eine nicht gezeigte Leitung mit der Erkennungseinrichtung 408 verbunden. Alternativ kann auch eine drahtlose Kommunikation zwischen Sensoreinheit 403 und Erkennungseinrichtung 408 vorgesehen sein.

Im Betrieb sendet die Sensoreinheit 403 ein Ultraschallsignal aus und empfängt dessen Reflektion. Die Erkennungseinrichtung 408 wertet das empfangene Signal aus und erkennt so, ob es sich bei der Quelle der Reflektion um eine Unwegsamkeit im Gelände handelt. Dabei wird das reflektierte Signal insbesondere auch von der Erkennungseinrichtung 408 derart ausgewertet, dass eine Charakterisierung der Unwegsamkeit möglich ist. Als Folge einer erkannten bzw. charakterisierten Unwegsamkeit liefert die Erkennungseinrichtung 408 ein entsprechendes Steuersignal an die zentrale Steuereinrichtung 60.

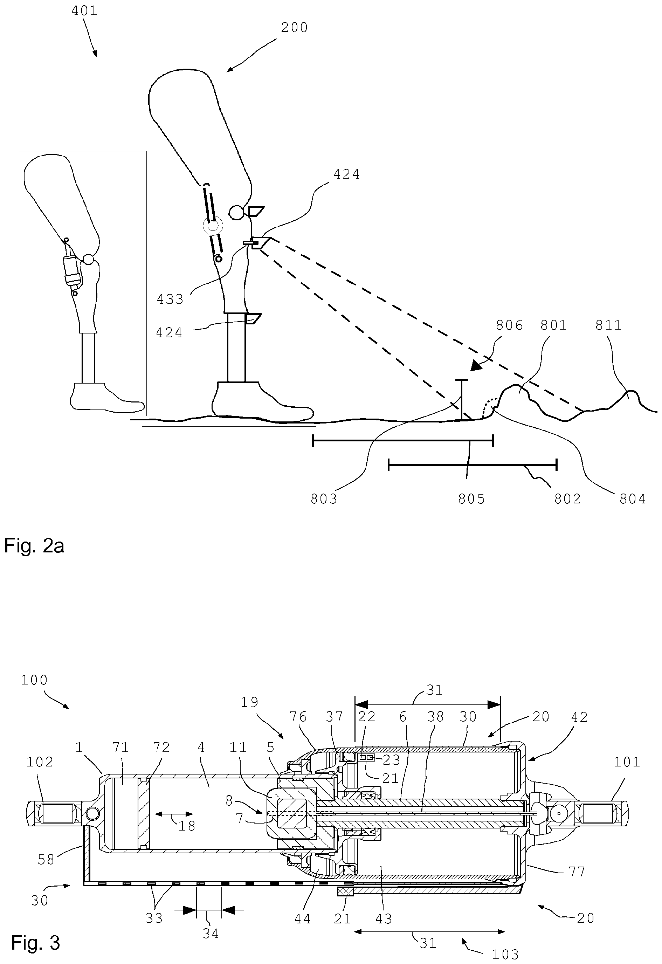

Die zentrale Steuereinrichtung 60 beeinflusst daraufhin ausgebildete erste Dämpfereinrichtung. Die Einstellung der Dämpfereinrichtung 100 durch die Steuereinrichtung 60 wird mit Bezug zu Figur 3 näher erläutert.

Die Bestimmung der Gehgeschwindigkeit kann auch über ein GPS-Signal erfolgen.

Figur 2 zeigt eine schematische Darstellung der Dämpfersteuerung 300 und der Kommunikationsverbindungen einiger beteiligter Komponenten. Die zentrale Steuereinrichtung 60 kann drahtgebunden oder drahtlos mit den einzelnen Komponenten verbunden sein. Beispielsweise kann die Steuereinrichtung 60 über WLAN, Bluetooth, ANT+, GPRS, UMTS, LTE oder sonstige Übertragungsstandards mit den anderen Komponenten verbunden sein. Gegebenenfalls kann die Steuereinrichtung 60 über die gepunktet dargestellte Verbindung mit dem Internet 53 drahtlos verbunden sein.

Die Steuereinrichtung 60 ist mit der Batterieeinheit 61 verbunden. Weiterhin kann die Steuereinrichtung 60 mit einer Detektoreinrichtung 20 oder mit mehreren Sensoren verbunden sein. Wenigstens zeitweise sind die Bedieneinrichtungen 150, nämlich die Betätigungseinrichtung 151 und die Einstelleinrichtung 152 drahtgebunden oder drahtlos mit der Steuereinrichtung 60 gekoppelt. Die Betätigungseinrichtung 151 ist vorzugsweise drahtgebunden an die Steuereinrichtung gekoppelt, kann aber auch drahtlos angebunden sein und über eine separate Batterie wie eine Knopfzelle oder dergleichen verfügen.

Die Steuereinrichtung 60 ist über Kabel, Netzwerkschnittstellen 54 oder Funknetzschnittstellen 55 mit Steuereinrichtungen 46 der Stoßdämpfer 100 an der Prothese verbunden. Die möglicherweise an jedem Stoßdämpfer 100 vorgesehene Steuereinrichtung 46 sorgt für die lokale Steuerung und kann jeweils eine Batterie aufweisen oder aber mit der zentralen Batterieeinheit 61 verbunden sein. Bevorzugt ist es, dass die Steuerung beider Stoßdämpfer über die Steuereinrichtung 60 erfolgt.

Vorzugsweise ist jedem Stoßdämpfer 100 wenigstens eine Detektoreinrichtung 20 zugeordnet, um Relativbewegungen zwischen den Komponenten 101 und 102 zu erfassen und insbesondere eine Relativposition der Komponenten 101 und 102 relativ zueinander zu bestimmen. Die Detektoreinrichtung 20 kann als Wegsensor ausgebildet sein oder einen solchen umfassen. Anhand der in der Speichereinrichtung 45 abgelegten Dämpferkennlinie 10 des Stoßdämpfers 100 wird nach Ermittlung eines Kennwerts für die Relativgeschwindigkeit die zugehörige Dämpfungskraft und eine passende Federkraft eingestellt. Eine passende Federkraft kann über das aktuelle Gewicht des Benutzers ermittelt werden.

In Figur 2 ist der Steuerkreislauf 12 schematisch dargestellt, der in der Speichereinrichtung 45 abgelegt ist und in der Steuereinrichtung 60 hinterlegt oder einprogrammiert ist. Der Steuerkreislauf 12 wird im Betrieb periodisch und insbesondere kontinuierlich periodisch durchgeführt. Im Schritt 52 wird mit der Detektoreinrichtung 20 eine aktuelle Relativbewegung bzw. Relativgeschwindigkeit der ersten Komponente 101 zur zweiten Komponente 102 erfasst. Aus den Werten der Detektoreinrichtung 20 oder der Sensoren wird im Schritt 52 ein Kennwert abgeleitet, der repräsentativ für die aktuelle Relativgeschwindigkeit ist.

Im nächsten Schritt 56 wird dann anschließend aus dem aktuellen bzw. ermittelten Kennwert unter Berücksichtigung der vorbestimmten oder ausgewählten Dämpferkennlinie die zugehörige einzustellende Dämpfungskraft abgeleitet. Daraus wird ein Maß für die aktuell einzustellende Feldstärke bzw. Stromstärke abgeleitet, mit welcher die einzustellende Dämpfungskraft wenigstens näherungsweise erreicht wird. Das Maß kann die Feldstärke selbst sein oder aber z. B. die Stromstärke angeben, mit welcher die einzustellende Dämpfungskraft wenigstens etwa erreicht wird.

Im darauf folgenden Schritt 70 wird die aktuell einzustellende Feldstärke erzeugt oder die entsprechende Stromstärke an die elektrische Spuleneinrichtung 11 als Felderzeugungseinrichtung angelegt, sodass innerhalb eines einzelnen Zyklus bzw. einer Zeitperiode des Steuerkreislaufes 12 die Dämpfungskraft erzeugt wird, die bei der gewählten oder vorbestimmten Dämpferkennlinie zu der aktuellen Relativgeschwindigkeit der ersten Komponente zu der zweiten Komponente vorgesehen ist. Anschließend startet der nächste Zyklus und es wird wieder Schritt 52 durchgeführt.

Die hier gezeigte zentrale Steuereinrichtung 60 ist zudem mit der erfindungsgemäßen Prothesen- oder Exoskelettkomponente 401 wirkverbunden. Die Prothesen- oder Exoskelettkomponente 401 besteht aus der Erkennungseinrichtung 408 und einem Ultraschallsensor 424. Der Ultraschallsensor 424 kann hier ein Ultraschallsignal aussenden und dieses Signal auch wieder empfangen. Der Sensor 424 vereint somit eine Sendeeinheit 413 und eine Empfangseinheit 423 in einem Bauteil. Dadurch ist eine besonders unauffällige und platzsparende Unterbringung möglich. Das ist besonders bei Sportprothesen von Vorteil, bei denen erhöhter Wert auf ein geringes Gewicht und gute aerodynamische Eigenschaften gelegt wird. Zudem wird auch das äußere Erscheinungsbild der Prothese 200 nicht beeinträchtigt.

Alternativ kann die Erkennungseinrichtung 408 auch mit einem Infrarotsensor 434 verbunden sein. Es kann auch ein Radarsensor 444 vorgesehen sein. Die Erkennungseinrichtung 408 weist hier auch eine integrierte Speichereinrichtung 418 auf. Damit ist eine Hinterlegung der erkannten Unwegsamkeiten (bzw. deren Daten) sowie der daraufhin vorgenommenen Dämpfereinstellungen möglich. Diese können dann später z. B. von einem Benutzer über eine entsprechende Schnittstelle wie z. B. ein Smartphone 160 abgerufen werden. Des Weiteren greift die Erkennungseinrichtung 408 hier auf Daten eines Sensormoduls 476 zurück, welches als Detektoreinrichtung 20 ausgebildet ist. Die Erkennungseinrichtung 408 berücksichtigt dabei die erfassten Werte der Detektoreinrichtung 20, um die Dämpferauslastung überwachen zu können.

Die Figur 2a zeigt die Prothese 200 der Figur 1 in einem stark schematisierten Gelände. Entlang der Gehstrecke befinden sich hier Unwegsamkeiten 801, 811, welche als Erhebungen bzw. Unebenheiten am Boden skizziert sind. Solche Unwegsamkeiten können beispielsweise sein: Steine, Stufen, Wurzeln, Senken, Bodenwellen, Schlaglöcher, Absätze, Erhebungen, Bordsteinkanten, Kopfsteinpflaster, Baumstümpfe, Äste und Baumstämme.

An der größer dargestellten Prothese 200 ist hier ein Ultraschallsensor 424 mittels einer Haltereinrichtung 433 angebracht. Dabei ist die Haltereinrichtung 433 so bemessen, dass sie nicht oder nur wenig über den Körper 111 hinaus nach vorne steht. Dadurch wird eine Beschädigung der Sensoreinheit 403 bei einem Anstoßen weitgehend vermieden. Bei der größer dargestellten Prothese 200 ist ein Drehdämpfer vorgesehen, der eine Dreh- oder Schwenkbewegung dämpft. Genauso bevorzugt ist der Einsatz eines Lineardämpfers, der an der schematisch dargestellten kleineren Prothese in Fig. 3 dargestellt ist.

Durch ein Verschwenken des Sensors 424 an der Haltereinrichtung 433 kann der Erfassungsbereich 806 des Sensors 424 optimal ausgerichtet werden. Eine solche Ausrichtung findet vorzugsweise einmalig bei der Installation der Prothesen- oder Exoskelettkomponente 401 statt. Möglich ist dabei auch, dass der Benutzer nach seinen Wünschen eine Ausrichtung des Erfassungsbereichs 806 selbst vornehmen kann.

Die hier gezeigten beispielhaften Anordnungen der Sensoreinheit 403 dienen der Veranschaulichung. Tatsächlich ist bevorzugt, dass an einer Prothese oder einem Exoskelett 200 lediglich eine Sensoreinheit 403 vorgesehen ist. Dabei kann die Sensoreinheit 403 jedoch mehrere Sensoren umfassen. Beispielsweise können in einer Sensoreinheit 403 vier oder sechs Ultraschallsensoren 424 vorgesehen sein, sodass die Auflösung verbessert bzw. der Erfassungsbereich 806 erweitert werden kann.

Bei der Verwendung eines Head up Sensors 403 wird diese und damit der Erfassungsbereich 806 in die Richtung geschwenkt, in welche der Benutzer schaut oder seinen Kopf wendet. Es kann aber auch eine Sensoreinheit 403 am Steuerrohr oder anderen Teilen vorgesehen sein, welche bei einer Kopfbewegung nicht verschwenkt werden. Die Ausrichtung der Sensoreinheit 403 zum Boden ist dabei je nach Typ und Bauart des Sensors festzulegen bzw. im Vorfeld zu ermitteln.

Möchte der Benutzer die Erkennungseinrichtung 408 nutzen, kann er diese über die Bedieneinrichtung 150 aktivieren. Die Erkennungseinrichtung 408 sendet dann über den Sensor 424 Ultraschallwellen in den Erfassungsbereich 806 aus. Ist der im Erfassungsbereich 806 liegende Geländeabschnitt frei von Unwegsamkeiten, wird das von der Erkennungseinrichtung 408 anhand der reflektierten Ultraschallwellen erkannt. Die Erkennungseinrichtung 408 nimmt dann keinerlei Veränderungen der Dämpfereinstellung vor. Die Dämpfereinrichtungen 1 sind dabei so eingestellt, wie es die Dämpfersteuerung 300 im Normalbetrieb vorsieht oder es den gewünschten Vorgaben des Benutzers entspricht.

Taucht nun im weiteren Verlauf eine Unwegsamkeit 801 im Erfassungsbereich 806 auf, kommt es zu einer veränderten Reflektion der Ultraschallwellen. Die Signalveränderung wird von der Erkennungseinrichtung 408 registriert und ausgewertet. Anhand der Auswertung kann insbesondere die Höhe 803 der Unwegsamkeit über dem Boden sowie die Entfernung 805 der Unwegsamkeit zur Vorderseite des Körpers oder der Komponente 200 bestimmt werden. Anhand der reflektierten Signale kann auch der Winkel 804 einer zur Prothese 200 zeigenden Fläche der Unwegsamkeit ermittelt werden. Möglich ist z. B. auch, dass die Form bzw. die dreidimensionale Geometrie der Unwegsamkeit wenigstens näherungsweise charakterisiert wird.

Anhand der Entfernung 805 ermittelt die Erkennungseinrichtung 803 den optimalen Zeitpunkt, um die Dämpfereinstellung für den zu erwartenden Stoß mit der Unwegsamkeit 801 einzustellen. Bis zum Erreichen der Unwegsamkeit 801 bleibt die Dämpfereinstellung vorzugsweise unverändert, damit die besten Fahreigenschaften für einen normalen bzw. ebenen Untergrund erzielt werden. Erreicht der vordere Punkt der Prothese nun die Unwegsamkeit 801, steuert die Erkennungseinrichtung 408 die zentrale Steuereinrichtung 60 an, sodass die Dämpfung in Richtung weich verstellt wird. Dabei können Parameter wie die Höhe 803 oder Winkel 804 herangezogen werden, um den Dämpfer genau um das Maß weicher einzustellen, wie es für eine derartige Unwegsamkeit optimal ist.

Erfolgt die Einstellung der Dämpfereinrichtung 1 beispielsweise über das Anlegen einer Feldstärke an ein magnetorheologisches Fluid 9, so kann aufgrund der besonders schnellen Reaktionszeit die Dämpferanpassung unmittelbar vor dem Auftreffen des Vorderrades 111 erfolgen. Die Dämpfereinrichtungen 1 mit derart kurzen Reaktionszeiten eignen sich besonders gut für die Verwendung mit der Erkennungseinrichtung 408, da der Erfassungsbereich 806 auf einen möglichst kurzen Nahbereich 802 fokussiert werden kann. Dadurch kann eine unerwünschte Erkennung von Unwegsamkeiten 801 vermieden werden, welche nach einer spontanen Ausweichbewegung gar nicht mehr relevant werden.

Je kürzer sich der Nahbereich 802 vor dem Benutzer erstreckt, desto wahrscheinlicher ist es, dass die erkannten Unwegsamkeiten 801 relevant werden und nicht etwa nach einem Richtungswechsel lediglich umgangen werden. Aufgrund der sehr kurzen Reaktionszeit der hier vorgestellten Dämpfereinstellung können beispielsweise Nahbereiche 802 realisiert werden, welche sich über eine Distanz erstrecken, die die Prothese oder das Exoskelett 200 in einer Sekunde oder sogar nur eine Zehntelsekunde zurücklegt. Die Einstellzeit der Dämpfereinrichtung 1 beträgt dabei vorzugsweise weniger als 10 Millisekunden. Dabei kann der Nahbereich 802, in welchem Unwegsamkeiten 801 erkannt werden und eine Dämpfereinstellung auslösen können, durch die Erkennungseinrichtung 408 dynamisch in Abhängigkeit der jeweiligen Geh- oder Laufgeschwindigkeit angepasst werden.

Ist die Unwegsamkeit 801 überwunden und befindet sich im Nahbereich 802 keine weitere Unwegsamkeit 811, so wird die Dämpfereinrichtung 1 wieder in die entsprechende Grundeinstellung für ebenes Gelände zurückgesetzt. Durch die Fokussierung des Erfassungsbereichs 806 auf einen sehr kurzen Nahbereich 802 werden außerhalb des Nahbereichs 802 liegende Unwegsamkeiten 811 nicht erfasst. Das ist jedoch keineswegs von Nachteil, da beim Gehen oder Wandern im Gelände häufige und schnelle Richtungswechsel auftreten. Daher ist es nicht unwahrscheinlich, dass weiter entfernt liegende Unwegsamkeiten 811 gar nicht relevant werden, sondern umgangen werden. Der kurze Nahbereich 802 hat somit den Vorteil, dass die Dämpfereinrichtung 1 auch genau auf den Untergrund eingestellt wird, welcher gerade aktuell ist.

Eine Unterscheidung z. B. zwischen einem Kopfsteinpflaster und einer Schotterstraße (Waldweg) ist mittels der Detektorsignale nach dem Stand der Technik (Wegsignal; Amplitude; Beschleunigungssignal) sehr schwierig, da die Signale der Detektoreinrichtungen auf beiden Untergründen sehr ähnlich sein können. Der Dämpfer sollte hierfür aber unterschiedlich eingestellt werden, was mit der Umfelderkennung möglich ist:
Bei Kopfsteinpflaster ist eher eine weichere Einstellung sinnvoll, damit kein Schläge auf den Körper weitergeleitet werden. Die Gefahr von plötzlichen Erhebungen/Löchern ist gering, daraus folgt, dass voraussichtlich über längere Phasen eine gleichbleibende Kennlinie vorliegt.

Auf einem Schotterweg wird die Dämpfung vorzugsweise eher härter eingestellt, da sonst die Prothese zu stark eintauchen kann und es instabile Zustände ergeben könnte. Daraus können instabile Zustände resultieren. Auch ist auf einem Waldweg oder auf einer Schotterstraße die Gefahr von plötzlichen größeren Steinen oder Unebenheiten erheblich größer, sodass auch ein schnellerer Wechsel von Kennlinien notwendig sein kann (höhere Taktrate der Elektronik).

Die hier gezeigte Erkennungseinrichtung 408 kommuniziert mit einer Detektoreinrichtung 20 der Dämpfereinrichtung 1 (vgl. Figur 3). Wie zuvor beschrieben ist diese Detektoreinrichtung 20 für die Ermittlung einer Relativgeschwindigkeit zweier sich zueinander bewegender Komponenten 101, 102 vorgesehen. Anhand der mit dieser Detektoreinrichtung 20 erfassten Relativgeschwindigkeit kann von der Erkennungseinrichtung 408 selbstständig überwacht werden, ob die vorgenommene Dämpfereinstellung für die überschrittene Unwegsamkeit 801 adäquat war oder nicht.

Überläuft die Prothese bzw. der Prothesenträger 200 beispielsweise eine Unwegsamkeit 801 und kommt es dabei zu einer nicht optimalen Auslastung der Dämpfereinrichtung 1, so erkennt die Erkennungseinrichtung 408 dies anhand der unpassenden Relativgeschwindigkeit der Dämpferkomponenten 101, 102. Tritt dann nachfolgend eine vergleichbare Unwegsamkeit 801 auf, nimmt die Überwachungseinrichtung 408 die Dämpfereinstellung unter Berücksichtigung eines geeigneten Korrekturfaktors vor. Ist die daraufhin gemessene Relativgeschwindigkeit der Dämpferkomponenten 101, 102 im Sollbereich, behält die Erkennungseinrichtung 408 den Korrekturfaktor bei. Befindet sich die Dämpferauslastung erneut außerhalb des Sollbereichs, passt die Erkennungseinrichtung 408 den Korrekturfaktor um ein bestimmtes Maß an.

Die Erkennungseinrichtung 408 ist hier mit einer Speichereinrichtung 418 ausgestattet, damit die Eigenschaften der erkannten Unwegsamkeit 801 und die daraufhin vorgenommenen Dämpfereinstellungen sowie mögliche Korrekturfaktoren hinterlegt werden können. Das ermöglicht einerseits eine besonders einfache Wartung und Kontrolle durch den Service, welcher die Speichereinrichtung 418 über eine geeignete Schnittstelle auslesen kann.

Zudem bietet es aber auch dem Träger hilfreiche Informationen, die er beispielsweise über sein Smartphone 160 aus der Speichereinrichtung 418 abrufen kann. Besonders bevorzugt werden die in der Speichereinrichtung 418 hinterlegten Informationen mit Positionsdaten verknüpft, welche beispielsweise aus einem GPSfähigen Smartphone 160 oder Smartdevice (wie z. B. eine Smartwatch) hinzugezogen werden können. Anhand dieser Daten kann der Benutzer in Verbindung mit digitalen Karten sehr detaillierte Streckenprofile erstellen, die anhand der gespeicherten Unwegsamkeiten ein sehr anschauliches Bild über die vorherrschenden Boden- bzw. Geländeverhältnisse bieten.

Es ist auch möglich, dass die Erkennungseinrichtung 408 zur Erkennung eines Sprungs des Prothesenträgers 200 ausgebildet ist. Ein Sprung kann beispielsweise dadurch erfasst werden, dass keine oder nur eine sehr geringe Reflexion auftritt oder dass ein Gewichtssensor im Schuh kein Gewicht erfasst. Eine derartige Erkennung von fehlendem Boden unter dem Schuh des Benutzers hat den Vorteil, dass die Dämpfereinrichtung 1 auf das Aufsetzen des Prothesenträgers 200 nach dem Sprung optimal eingestellt werden kann. Zur Erkennung, ob der Prothesenträger 200 nach dem Sprung zuerst mit dem linken oder rechten Bein aufsetzt, kann die Erkennungseinrichtung 408 wenigstens einen Lagesensor oder dergleichen aufweisen.

In einer anderen Ausgestaltung ist die Sensoreinheit 403 vorzugsweise mit einer als Kamera ausgebildeten Empfangseinheit 423 ausgestattet. Mit einer solchen Empfangseinheit 423 werden optische Projektionen der Unwegsamkeit erfasst und zur Erkennung von Unwegsamkeiten durch die Erkennungseinrichtung 408 herangezogen. Eine Sendeeinheit 413 ist dabei nicht erforderlich und kann weggelassen werden.

Es können auch zwei oder mehr als Kamera ausgebildete Empfangseinheiten 423 bzw. wenigstens eine Stereokamera vorgesehen sein, sodass optische Projektionen mit dreidimensionaler bzw. räumlicher Information ableitbar sind. Dadurch können Abstand, Form und Größe der Unwegsamkeit besonders detailliert und zuverlässig bestimmt werden.

Die Sensoreinheit 403 kann auch eine Kamera mit einer Lichtquelle umfassen und als Triangulationseinrichtung ausgebildet sein. Dabei projiziert die Lichtquelle ein definiertes Muster auf die Unwegsamkeit und die Kamera nimmt dieses Muster aus mehreren Blickwinkeln auf und berechnet aus der Musterverzerrung die Form oder Größe der Unwegsamkeit.

Möglich ist auch, dass die Sensoreinheit 403 mittels einer Lichtquelle Licht aussendet und die Erkennungseinrichtung 408 anhand einer Laufzeitmessung die Entfernung der Unwegsamkeit ermittelt.

Figur 3 zeigt ein Ausführungsbeispiel einer Stoßdämpfereinrichtung 100 für eine Prothesen- oder Exoskelettkomponente 401 mit einer Dämpfereinrichtung 1 und hier einer Federeinrichtung 42, die als Luftfeder ausgeführt ist und eine Positivkammer 43 und eine Negativkammer 44 umfasst. Der Dämpfereinrichtung 1 wird mit dem ersten Ende als Anschlusseinheit bzw. Komponente 101 und dem zweiten Ende als Anschlusseinheit bzw. Komponente 102 an unterschiedlichen Teilen der Komponente 401 aus Fig. 1 befestigt, um Relativbewegungen zu dämpfen. Die Dämpfereinrichtung 1 umfasst ein Dämpfergehäuse 2 und eine erste Dämpferkammer 3 und eine zweite Dämpferkammer 4, die durch das als Kolben 5 ausgebildete Dämpfungsventil 8 voneinander getrennt sind. In anderen Ausgestaltungen ist auch ein externes Dämpferventil 8 möglich, welches außerhalb des Dämpfergehäuses 2 angeordnet und über entsprechende Zuleitungen angeschlossen ist.

Der Kolben 5 ist mit einer Kolbenstange 6 verbunden. In dem Dämpfungskolben 5 ist das gestrichelt eingezeichnete magnetorheologische Dämpfungsventil 8 vorgesehen, welches hier eine elektrische Spule 11 als Felderzeugungseinrichtung umfasst, um eine entsprechende Feldstärke zu erzeugen. Das Dämpfungsventil 8 bzw. der "Öffnungszustand" des Dämpfungsventils wird über die elektrische Spuleneinrichtung 11 angesteuert.

Die Spule der elektrischen Spuleneinrichtung 11 ist nicht in Umfangsrichtung um die Kolbenstange 6 herumgewickelt, sondern um eine Achse, die sich quer zur Längserstreckung der Kolbenstange 6 (und hier parallel zur Zeichnungsebene) erstreckt. Eine Relativbewegung findet hier linear statt und erfolgt in der Bewegungsrichtung 18. Die Magnetfeldlinien verlaufen dabei in dem Zentralbereich des Kerns etwa senkrecht zur Längserstreckung der Kolbenstange 6 und durchtreten somit etwa senkrecht die Dämpfungskanäle 7. Ein Dämpfungskanal befindet sich hinter der Zeichnungsebene und ist gestrichelt eingezeichnet. Dadurch wird eine effektive Beeinflussung des sich in den Dämpfungskanälen 7 befindenden magnetorheologischen Fluides bewirkt, sodass der Durchfluss durch das Dämpfungsventil 8 effektiv gedämpft werden kann.

In dem Dämpfergehäuse 2 ist ein Ausgleichskolben 72 angeordnet, der einen vorzugsweise mit einem Gas gefüllten Ausgleichsraum 71 für das Volumen der beim Einfedern eintretenden Kolbenstange abtrennt.

Nicht nur in dem Dämpfungsventil 8, sondern hier in den beiden Dämpfungskammern 3 und 4 befindet sich hier überall (außer im Ausgleichsraum 71) ein magnetorheologisches Fluid als feldempfindliches Medium.

Die Stoßdämpfereinrichtung 100 weist eine Detektoreinrichtung 20 auf. Die Detektoreinrichtung 20 umfasst jeweils einen Detektorkopf 21 und eine strukturiert ausgebildete Maßstabeinrichtung 30.

Die Maßstabeinrichtung 30 umfasst hier ein Sensorband mit Permanentmagneteinheiten als Felderzeugungseinheiten. Die Pole der Permanentmagneteinheiten wechselnd sich alternierend ab, sodass entlang der Bewegungsrichtung des Detektors 22 alternierend Nord-Südpole angeordnet sind. Die magnetische Feldstärke wird durch den Detektorkopf ausgewertet und daraus wird die jeweils aktuelle Position 19 ermittelt.

Die Federeinrichtung 42 erstreckt sich hier wenigstens teilweise um die Dämpfereinrichtung 1 herum und umfasst ein Federgehäuse 76. Ein Ende des Dämpfers 1 ist mit einem Federungskolben 37 verbunden bzw. bildet einen solchen. Der Federungskolben 37 trennt die Positivkammer 43 von einer Negativkammer 44.

Das Federgehäuse 76 wird zu dem Ende der Anschlusseinheit 101 durch einen Deckel 77 abgeschlossen. Dort wird auch das Anschlusskabel 38 für die elektrische Spuleneinrichtung 11 herausgeführt. Vorzugsweise wird dort auch ein elektrisches Anschlusskabel für die Detektoreinrichtung 20 nach außen herausgeführt.

Zur besseren Verdeutlichung sind in Fig. 3 zwei verschiedene Varianten einer Detektoreinrichtung 20 eingezeichnet.

Die Detektoreinrichtung 20 umfasst zwei Sensorteile, nämlich den Detektorkopf 21, der in der oberhalb der Mittellinie dargestellten Variante innerhalb der Positivkammer 43 der Federeinrichtung 42 angeordnet ist. Die Detektoreinrichtung 20 umfasst als weiteres Sensorteil die Maßstabeinrichtung 30, die in dieser Variante an dem Federgehäuse 76 angeordnet oder aufgenommen ist. Je nach Ausgestaltung und Materialwahl des Federgehäuses 76 und nach Messprinzip der Detektoreinrichtung 20 kann die Maßstabeinrichtung 30 in die Wandung des Federgehäuses 76 integriert sein oder aber auf der Innenwandung des Federgehäuses 76 angeordnet werden oder auch außen auf dem Federgehäuse 76 angebracht oder aufgebracht sein.

Der Detektorkopf 21 umfasst zwei Detektoren 22 und 23, die hier in Bewegungsrichtung 18 versetzt zueinander angeordnet sind.

Die Maßstabeinrichtung 30 weist eine sich über eine Messstrecke 31 erstreckende Struktur 32 auf, über der sich die physikalischen Eigenschaften der Maßstabeinrichtung 30 periodisch ändern.

Vorzugsweise sind auf der Maßstabeinrichtung 30 Sensorabschnitte 33 angeordnet, die sich jeweils wiederholende elektrische und/oder magnetische Eigenschaften haben und somit die Struktur 32 der Maßstabeinrichtung 30 bilden.

Findet nun eine Relativbewegung der Anschlusseinheiten 101 und 102 des Dämpfers 1 zueinander statt, so ändert sich die Position 19 des Dämpfers 1 und es verschiebt sich die relative Position des Detektorkopfs 21 relativ zu der Maßstabeinrichtung 30. Durch Auswertung der Signalstärke eines Detektors 22, 23 und insbesondere von wenigstens zwei Detektoren 22, 23 kann somit auf die relative Position des Detektorkopfs 21 relativ zu einem Sensorabschnitt 33 bzw. gegenüber der Maßstabeinrichtung 30 oder der absoluten Position innerhalb eines Sensorabschnitts 33 zurückgeschlossen werden. Die Sensorabschnitte 33 haben eine Länge 34, die konstant oder auch variabel sein kann. Werden zwei Detektoren in Bewegungsrichtung 18 versetzt zueinander angeordnet und erfassen beide Detektoren das Magnetfeld der Maßstabeinrichtung 30, so kann durch Auswertung der Signale die Position 19 und die Bewegungsrichtung 18 sehr genau ermittelt werden.

Bei der kontinuierlichen Bewegung wird in der Speichereinrichtung 45 der Steuereinrichtung 46 die Anzahl der passierten Sensorabschnitte bzw. Perioden hinterlegt, sodass auf die absolute Position 19 zurückgeschlossen werden kann. Dazu ist nur erforderlich, dass die Messfrequenz derart hoch ist, dass nicht während eines Messzyklus ein vollständiger Sensorabschnitt "unbemerkt" vorbeigeschoben wird.

Alternativ zu der in Figur 3 oberhalb der Symmetrielinie der Dämpfereinrichtung 1 eingezeichneten Variante ist unterhalb der Symmetrielinie eine Alternative des Detektors 20 zusätzlich dargestellt, bei der der Detektoreinrichtung 20 vollständig außerhalb des Dämpfergehäuses 2 und des Federgehäuses 76 angeordnet ist. Ein Halter 58 hält die Maßstabeinrichtung 30 und verbindet die Maßstabeinrichtung fest mit einem Ende bzw. einer Anschlusseinheit 102 der Stoßdämpfereinrichtung 100. Mit dem anderen Ende bzw. der anderen Anschlusseinheit 101 der Stoßdämpfereinrichtung 100 ist der Detektorkopf 21 verbunden. Der Detektorkopf 21 wird so gehalten, dass er berührungslos mit einem geringen Abstand von der Maßstabeinrichtung 30 angeordnet ist. Bei einer Relativbewegung der Anschlusseinheiten des Stoßdämpfers 100 erfolgt so auch eine Relativbewegung der Maßstabeinrichtung 30 relativ zu dem Detektorkopf 21. Auch hier kann über die Messstrecke 31, die vorzugsweise im Wesentlichen dem Dämpferhub 103 entspricht, über die Auswertung der Feldstärken eine Relativposition ermittelt werden.

**Bezugszeichenliste:**

| | | | |
|---|---|---|---|
| 1 | Dämpfereinrichtung | 61 | Batterieeinheit |
| 2 | Dämpfergehäuse | 70 | Schritt |
| 3 | erste Dämpferkammer | 71 | Ausgleichsraum |
| 4 | zweite Dämpferkammer | 72 | Ausgleichskolben |
| 5 | Dämpfungskolben | 76 | Federgehäuse |
| 6 | Kolbenstange | 77 | Deckel |
| 7 | Dämpfungskanal | 90 | Dämpferkennlinie |
| 8 | Dämpfungsventil | 100 | Stoßdämpfer |
| 9 | MRF | 101,102 | Komponente |
| 10 | Dämpferkennlinie | 118 | Winkelsensor |
| 11 | elektrische Spule | 150 | Bedieneinrichtung |
| 12 | Steuerkreislauf | 151 | Betätigungseinrichtung |
| 18 | Bewegungsrichtung | 152 | Einstelleinrichtung |
| 19 | Position | 153 | Eingabeeinheit |
| 20 | Detektoreinrichtung | 160 | Smartphone |
| 21, 22 | Detektor | 161-162 | Bereich |
| 30 | Maßstabeinrichtung | 190 | Dämpferkennlinie |
| 31 | Messstrecke | 200 | Prothese, Exoskelett |
| 32 | Struktur | 401 | Prothesen- oder Exoskelettkomponente |
| 33 | Sensorabschnitt | | |
| 37 | Federungskolben | 403 | Sensoreinheit |
| 38 | Kabel | 408 | Erkennungseinrichtung |
| 42 | Federeinrichtung | 413 | Sendeeinheit |
| 43 | Positivkammer | 418 | Speichereinrichtung |
| 44 | Negativkammer | 423 | Empfangseinheit |
| 42 | Isoliermaterial | 424 | Ultraschallsensor |
| 45 | Speichereinrichtung | 433 | Halteeinrichtung |
| 46 | Steuereinrichtung | 434 | Infrarotsensor |
| 48 | Daten | 444 | Radarsensor |
| 49 | Display, Anzeige | 476 | Sensormodul |
| 52 | Schritt | 801 | Unwegsamkeit |
| 53 | Internet | 802 | Nahbereich |
| 54 | Netzwerkschnittstelle | 803 | Höhe |
| 55 | Funknetzschnittstelle | 804 | Winkel |
| 56 | Schritt | 805 | Entfernung |
| 57 | Touchscreen | 806 | Erfassungsbereich |
| 58 | Halter | 811 | Unwegsamkeit |
| 60 | Steuereinrichtung | | |

## Patentansprüche

1. Prothesen- oder Exoskelettkomponente (401) für eine Prothese oder ein Exoskelett (200) mit wenigstens einer Stoßdämpfereinrichtung (100) mit wenigstens einer über wenigstens eine Steuereinrichtung (60) steuerbaren Dämpfereinrichtung (1),
wobei wenigstens eine Erkennungseinrichtung (408) mit wenigstens einer Sensoreinheit (403) vorgesehen ist, welche wenigstens eine Empfangseinheit (423) zur berührungslosen Erfassung wenigstens eines von wenigstens einer Unwegsamkeit (801) beeinflussten Signals umfasst
und dass die Erkennungseinrichtung (408) dazu geeignet und ausgebildet ist, in Abhängigkeit des erfassten Signals die Unwegsamkeit (801) zu erkennen und wenigstens eine Dämpfereinrichtung (100) in Abhängigkeit der erkannten Unwegsamkeit (801) zu steuern, sodass wenigstens eine Dämpfungseigenschaft der Dämpfereinrichtung (100) durch ein Signal der Erkennungseinrichtung (408) einstellbar ist,
**dadurch gekennzeichnet,**
**dass** die Sensoreinheit (403) wenigstens eine Sendeeinheit (413) zur Aussendung wenigstens eines Signals umfasst und wobei die Empfangseinheit (423) dazu geeignet und ausgebildet ist, wenigstens eine von der Unwegsamkeit (801) ausgehende Reflexion des ausgesendeten Signals als Signal zu erfassen.

2. Prothesen oder Exoskelettkomponente (401) nach Anspruch 1, wobei die Dämpfereinrichtung (1) wenigstens eine erste Dämpferkammer (3) und wenigstens eine zweite Dämpferkammer (4) umfasst, welche über wenigstens ein steuerbares Dämpfungsventil (8) miteinander gekoppelt sind und wobei dem Dämpfungsventil (8) wenigstens eine durch die Erkennungseinrichtung (408) steuerbare Felderzeugungseinrichtung (11) zugeordnet ist, welche zur Erzeugung und Steuerung einer Feldstärke in wenigstens einem Dämpfungskanal (7) des Dämpfungsventils (8) dient, wobei in dem Dämpfungskanal (7) ein feldempfindliches rheologisches Medium (9) vorgesehen ist.

3. Prothesen oder Exoskelettkomponente (401) nach einem der vorhergehenden Ansprüche, wobei die Erkennungseinrichtung (408) dazu geeignet und ausgebildet ist, für die Steuerung der Dämpfereinrichtung (1) nur Unwegsamkeiten (801) in einem vorgegebenen Nahbereich (802) zu berücksichtigen, wobei vorzugsweise die Erkennungseinrichtung (408) dazu geeignet und ausgebildet ist, den Nahbereich (802) in Abhängigkeit einer Gehgeschwindigkeit der Prothese (200) vorzugeben und wobei sich der Nahbereich (802) über die Distanz erstreckt, welche der Prothesenträger (200) anhand der Gehgeschwindigkeit in einer Sekunde zurücklegt.

4. Prothesen oder Exoskelettkomponente (401) nach einem der vorhergehenden Ansprüche, wobei die Erkennungseinrichtung (408) dazu geeignet und ausgebildet ist, infolge einer erkannten Unwegsamkeit (801) im Nahbereich (802) die Dämpfungseigenschaft der Dämpfereinrichtung (1) in weniger als 30 ms Sekunden einzustellen.

5. Prothesen oder Exoskelettkomponente (401) nach einem der vorhergehenden Ansprüche, wobei die Erkennungseinrichtung (408) dazu geeignet und ausgebildet ist, die Höhe der Unwegsamkeit (801) über dem Boden und/oder den Winkel wenigstens eines Bereichs der Unwegsamkeit (801) zum Boden zu ermitteln und für die Steuerung der Dämpfereinrichtung (1) zu berücksichtigen.

6. Prothesen- oder Exoskelettkomponente (401) nach einem der vorhergehenden Ansprüche, wobei die Steuereinrichtung (60) geeignet und ausgebildet ist, die der Dämpfereinrichtung (1) bis zum Erreichen der Unwegsamkeit härter einzustellen.

7. Prothesen oder Exoskelettkomponente (401) nach einem der vorhergehenden Ansprüche, wobei die Erkennungseinrichtung (408) dazu geeignet und ausgebildet ist, bei der Steuerung der Dämpfereinrichtung (1) wenigstens einen voreingestellten Grenzwert für eine maximale und/oder minimale Dämpfung zu berücksichtigen.

8. Prothesen oder Exoskelettkomponente (401) nach einem der vorhergehenden Ansprüche, wobei die Stoßdämpfereinrichtung (1) wenigstens eine einem Knie (111) zugeordnete erste Dämpfereinrichtung (114) und wenigstens eine einer Hüfte (112) zugeordnete zweite Dämpfereinrichtung (115) umfasst und wobei die Erkennungseinrichtung (408) dazu geeignet und ausgebildet ist, die zweite Dämpfereinrichtung (115) zeitverzögert zu der ersten Dämpfereinrichtung (114) einzustellen.

9. Prothesen oder Exoskelettkomponente (401) nach einem der vorhergehenden Ansprüche, wobei der Dämpfereinrichtung (1) wenigstens ein Sensormodul (415) zur Erfassung einer Dämpferauslastung zugeordnet ist und wobei die Erkennungseinrichtung (408) dazu geeignet und ausgebildet ist, das Sensormodul (476) auszulesen und die Dämpferauslastung als Folge einer auf eine erkannte Unwegsamkeit (801) erfolgten Einstellung der Dämpfereigenschaft zu registrieren und bei einer Abweichung der registrierten Dämpferauslastung von einem vorgegebenen Maß für die Dämpferauslastung die Steuerung der Dämpfereinrichtung (1) anzupassen.

10. Prothesen oder Exoskelettkomponente (401) nach einem der vorhergehenden Ansprüche, wobei die Erkennungseinrichtung (408) wenigstens eine Speichereinrichtung (418) zur Aufzeichnung der erkannten Unwegsamkeiten (801) umfasst.

11. Prothesen oder Exoskelettkomponente (401) nach einem der vorhergehenden Ansprüche, wobei die Sensoreinheit (403) an Kopf angeordnet ist, welcher verschwenkt.

12. Prothesen oder Exoskelettkomponente (401) nach einem der vorhergehenden Ansprüche, wobei die Sensoreinheit (403) verschwenkbar an wenigstens einer Halteeinrichtung (433) aufgenommen ist, sodass der Sendewinkel und/oder Empfangswinkel zum Boden einstellbar ist und/oder wobei die Sensoreinheit (403) als ein Ultraschallsensor (424) und/oder als ein Infrarotsensor (434) und/oder als ein Radarsensor (444) ausgebildet ist.

13. Prothesen oder Exoskelettkomponente (401) nach einem der vorhergehenden Ansprüche, wobei die Erkennungseinrichtung (408) dazu geeignet und ausgebildet ist, wenigstens zwei Stoßdämpfereinrichtungen zu steuern.

14. Verfahren zum Betreiben einer Prothesen- oder Exoskelettkomponente (401) einer Prothese oder eines Exoskeletts(200), welches wenigstens eine Stoßdämpfereinrichtung (100) mit wenigstens einer Dämpfereinrichtung (1) aufweist, wobei die Dämpfereinrichtung (1) über wenigstens eine Steuereinrichtung (60) gesteuert wird,
wobei mittels einer Erkennungseinrichtung (408) wenigstens ein Signal berührungslos erfasst wird und dass anhand des berührungslos erfassten Signals die Unwegsamkeit (801) erkannt wird und in Abhängigkeit der erkannten Unwegsamkeit (801) die Dämpfereinrichtung (1) gesteuert und wenigstens eine Dämpfungseigenschaft der Dämpfereinrichtung (1) eingestellt wird,
**dadurch gekennzeichnet,**
**dass** mittels der Erkennungseinrichtung (408) wenigstens ein Signal ausgesendet und wenigstens eine von der Unwegsamkeit (801) ausgehende Reflexion des ausgesendeten Signals empfangen und als Signal erfasst wird.

15. Verfahren nach dem vorhergehenden Anspruch, wobei nach Erkennung der Unwegsamkeit bis zum Erreichen der Unwegsamkeit die Dämpfereinrichtung (1) härter eingestellt wird.

## Claims

1. Prosthesis or exoskeleton component (401) for a prosthesis or an exoskeleton (200) having at least one shock absorber device (100) having at least one damper device (1) that can be controlled by at least one control device (60),
wherein at least one identification device (408) with at least one sensor unit (403) is provided which comprises at least one receiving unit (423) for non-contact capturing of at least one signal that is influenced by at least one hazard (801),
and that the identification device (408) is suitable and configured to identify a hazard (801) in dependence on the captured signal and to control at least one damper device (100) in dependence on the identified hazard (801) so that at least one damping characteristic of the damper device (100) can be adjusted by a signal from the identification device (408),
**characterised in**
**that** the sensor unit (403) comprises at least one transmitting unit (413) for emitting at least one signal and wherein the receiving unit (423) is suitable and configured to capture as a signal at least one reflection of the emitted signal starting from the hazard (801).

2. The prosthesis or exoskeleton component (401) according to claim 1 wherein the damper device (1) comprises at least one first damper chamber (3) and at least one second damper chamber (4) which are coupled to one another by means of at least one controllable damping valve (8) and wherein at least one field generating device (11) that can be controlled by the identification device (408) is associated to the damping valve (8) which serves to generate and control a field intensity in at least one damping duct (7) of the damping valve (8) wherein a field-sensitive rheological medium (9) is provided in the damping duct (7).

3. The prosthesis or exoskeleton component (401) according to any of the preceding claims wherein the identification device (408) is suitable and configured to take only hazards (801) in a specified near zone (802) into account for controlling the damper device (1), wherein the identification device (408) is preferably suitable and configured to specify the near zone (802) in dependence on a walking speed of the prosthesis (200) and wherein the near zone (802) extends over the distance which the prosthesis wearer (200) travels in one second according to the walking speed.

4. The prosthesis or exoskeleton component (401) according to any of the preceding claims wherein the identification device (408) is suitable and configured to adjust the damping characteristic of the damper device (1) in under 30 ms seconds due to an identified hazard (801) in the near zone (802).

5. The prosthesis or exoskeleton component (401) according to any of the preceding claims wherein the identification device (408) is suitable and configured to determine the height of the hazard (801) above the ground and/or the angle of at least one portion of the hazard (801) relative to the ground and to take these into account for controlling the damper device (1).

6. The prosthesis or exoskeleton component (401) according to any of the preceding claims wherein the control device (60) is suitable and configured to set the damper device (1) harder until the hazard is reached.

7. The prosthesis or exoskeleton component (401) according to any of the preceding claims wherein the identification device (408) is suitable and configured to take into account at least one preset threshold value for a damping maximum and/or minimum for controlling the damper device (1).

8. The prosthesis or exoskeleton component (401) according to any of the preceding claims wherein the shock absorber device (1) comprises at least one first damper device (114) assigned to a knee (111) and at least one second damper device (115) assigned to a hip (112) and wherein the identification device (408) is suitable and configured to adjust the second damper device (115) with a time delay relative to the first damper device (114).

9. The prosthesis or exoskeleton component (401) according to any of the preceding claims wherein the damper device (1) has at least one sensor module (415) assigned to it for capturing a damper utilization and wherein the identification device (408) is suitable and configured to read out the sensor module (476) and to register the damper utilization resulting from adjustment of the damper properties made due to an identified hazard (801) and, in case that the registered damper utilization deviates from a specified amount for the damper utilization, to adapt the control of the damper device (1).

10. The prosthesis or exoskeleton component (401) according to any of the preceding claims wherein the identification device (408) comprises at least one memory device (418) for recording the identified hazards (801).

11. The prosthesis or exoskeleton component (401) according to any of the preceding claims wherein the sensor unit (403) is disposed on a head which is pivotable.

12. The prosthesis or exoskeleton component (401) according to any of the preceding claims wherein the sensor device (403) is pivotably accommodated on at least one holding device (433) so that the transmitting angle and/or receiving angle relative to the ground is adjustable and/or wherein the sensor unit (403) is configured as an ultrasonic sensor (424) and/or an infrared sensor (434) and/or a radar sensor (444).

13. The prosthesis or exoskeleton component (401) according to any of the preceding claims wherein the identification device (408) is suitable and configured to control at least two shock absorber devices.

14. Method for operating a prosthesis or exoskeleton component (401) of a prosthesis or of an exoskeleton (200) comprising at least one shock absorber device (100) having at least one damper device (1) wherein the damper device (1) is controlled by at least one control device (60),
wherein at least one signal is captured contactless by means of an identification device (408) and that the hazard (801) is identified by way of the signal captured contactless and the damper device (1) is controlled in dependence on the identified hazard (801) and at least one damping characteristic of the damper device (1) is adjusted,
**characterised in**
**that** at least one signal is emitted by means of the identification device (408) and at least one reflection of the emitted signal coming from the hazard (801) is received and captured as a signal.

15. The method according to the preceding claim wherein the damper device (1) is set harder after identifying the hazard until the hazard is reached.

## Revendications

1. Composante de prothèse ou d'exosquelette (401) pour une prothèse ou un exosquelette (200), dotée d'au moins un moyen antichoc (100) avec au moins un dispositif amortisseur (1) susceptible d'être commandé par un organe de commande (60),
au moins un système de détection (408) doté d'au moins une unité de détection (403) étant prévu, lequel comprend au moins une unité de réception (423) pour acquérir sans contact au moins un signal affecté par au moins une impraticabilité (801),
et que ledit système de détection (408) est apte à et conçu pour détecter ladite impraticabilité (801) en fonction du signal acquis, et pour commander au moins un dispositif amortisseur (100) en fonction de ladite impraticabilité (801) détectée, de sorte qu'au moins une propriété d'amortissement dudit dispositif amortisseur (100) est susceptible d'être paramétrée via un signal émis par ledit système de détection (408),
**caractérisé en ce**
**que** ladite unité de détection (403) comprend au moins une unité émettrice (413) pour envoyer au moins un signal, et l'unité réceptrice (423) étant apte à et conçue pour acquérir en tant que signal au moins une réflexion du signal envoyé en provenance de ladite impraticabilité (801).

2. Composante de prothèse ou d'exosquelette (401) selon la revendication 1, le dispositif amortisseur (1) comprenant au moins une première chambre d'amortisseur (3) et au moins une deuxième chambre d'amortisseur (4) qui sont accouplées l'une à l'autre via au moins une soupape d'amortissement (8) susceptible d'être commandée, et au moins un système de génération de champ (11), susceptible d'être commandé par le système de détection (408), étant associé à ladite soupape d'amortissement (8), lequel système de génération de champ sert à générer et à commander une intensité de champ dans au moins un canal d'amortissement (7) de ladite soupape d'amortissement (8), un milieu (9) rhéologique sensible au champ étant prévu dans ledit canal d'amortissement (7).

3. Composante de prothèse ou d'exosquelette (401) selon l'une quelconque des revendications précédentes, le système de détection (408) étant apte à et conçu pour, dans la commande du dispositif amortisseur (1), ne prendre en compte que des impraticabilités (801) dans une zone de proximité (802) prédéterminée, ledit système de détection (408) de préférence étant apte à et conçu pour prédéterminer ladite zone de proximité (802) en fonction d'une vitesse de marche de la prothèse (200), et ladite zone de proximité (802) s'étendant sur la distance parcourue par le porteur de la prothèse (200) en une seconde sur la base de la vitesse de marche.

4. Composante de prothèse ou d'exosquelette (401) selon l'une quelconque des revendications précédentes, le système de détection (408) étant apte à et conçu pour paramétrer en moins de 30 ms secondes la propriété d'amortissement du dispositif amortisseur (1) suite à la détection d'une impraticabilité (801) dans la zone de proximité (802).

5. Composante de prothèse ou d'exosquelette (401) selon l'une quelconque des revendications précédentes, le système de détection (408) étant apte à et conçu pour déterminer et pour prendre en compte dans la commande du dispositif amortisseur (1) la hauteur de l'impraticabilité (801) au-dessus du sol et/ou l'angle d'au moins une zone de l'impraticabilité (801) par rapport au sol.

6. Composante de prothèse ou d'exosquelette (401) selon l'une quelconque des revendications précédentes, l'organe de commande (60) étant apte à et conçu pour paramétrer la dureté de celle du dispositif amortisseur (1) à un niveau plus élevé jusqu'à ce que l'impraticabilité soit atteinte.

7. Composante de prothèse ou d'exosquelette (401) selon l'une quelconque des revendications précédentes, le système de détection (408) étant apte à et conçu pour prendre en compte, dans la commande du dispositif amortisseur (1), au moins un seuil paramétré au préalable pour un amortissement maximum et/ou minimum.

8. Composante de prothèse ou d'exosquelette (401) selon l'une quelconque des revendications précédentes, le moyen antichoc (1) comprenant au moins un premier dispositif amortisseur (114) associé à un genou (111) et au moins un deuxième dispositif amortisseur (115) associé à une hanche (112), et le système de détection (408) étant apte à et conçu pour paramétrer le deuxième dispositif amortisseur (115) en différé par rapport au premier dispositif amortisseur (114).

9. Composante de prothèse ou d'exosquelette (401) selon l'une quelconque des revendications précédentes, au moins un module capteur (415) destiné à acquérir une charge d'amortissement étant associé au dispositif amortisseur (1), et le système de détection (408) étant apte à et conçu pour lire ledit module capteur (476) et pour consigner la charge d'amortissement faisant suite à un paramétrage de la propriété d'amortissement dû à une impraticabilité (801) détectée, et pour ajuster la commande du dispositif amortisseur (1) si la charge d'amortissement consignée s'écarte d'une mesure prédéterminée pour la charge d'amortissement.

10. Composante de prothèse ou d'exosquelette (401) selon l'une quelconque des revendications précédentes, le système de détection (408) comprenant au moins un dispositif d'enregistrement (418) pour enregistrer les impraticabilités (801) détectées.

11. Composante de prothèse ou d'exosquelette (401) selon l'une quelconque des revendications précédentes, l'unité de détection (403) étant agencée à la tête, laquelle pivote.

12. Composante de prothèse ou d'exosquelette (401) selon l'une quelconque des revendications précédentes, l'unité de détection (403) étant montée pivotante sur au moins un système de fixation (433), de sorte que l'angle d'émission et/ou l'angle de réception par rapport au sol sont susceptibles d'être paramétrés et/ou l'unité de détection (403) étant constituée en tant que capteur ultrasonique (424) et/ou en tant que capteur infrarouge (434) et/ou en tant que capteur radar (444).

13. Composante de prothèse ou d'exosquelette (401) selon l'une quelconque des revendications précédentes, le système de détection (408) étant apte à et conçu pour commander au moins deux moyens antichoc.

14. Procédé d'exploitation d'une composante de prothèse ou d'exosquelette (401) d'une prothèse ou d'un exosquelette (200), qui présente au moins un moyen antichoc (100) doté d'au moins un dispositif amortisseur (1), ledit dispositif amortisseur (1) étant commandé via au moins un organe de commande (60),
à l'aide d'un système de détection (408) au moins un signal étant acquis sans contact et que sur la base dudit signal acquis sans contact, l'impraticabilité (801) est détectée, et en fonction de ladite impraticabilité (801) détectée, le dispositif amortisseur (1) est commandé et au moins une propriété d'amortissement dudit dispositif amortisseur (1) est paramétrée,
**caractérisé en ce**
**que**, à l'aide du système de détection (408) au moins un signal est envoyé et au moins une réflexion du signal envoyé, en provenance de l'impraticabilité (801), est reçue et acquise en tant que signal.

15. Procédé selon la revendication précédente, le dispositif amortisseur (1) étant paramétré à un niveau de dureté plus élevé une fois que l'impraticabilité a été détectée et jusqu'à ce que ladite impraticabilité soit atteinte.
